# EUROPEAN PATENT SPECIFICATION

(11) **EP 4 346 779 B1**
(45) Date of publication and mention of the grant of the patent: **11.11.2026**
(21) Application number: 22729653.0
(22) Date of filing: 19.05.2022
(51) Int. Cl.: A61K 9/48

(54) **HARD SHELL CAPSULES WITH MODIFIED RELEASE COATING**
HARTSCHALENKAPSELN MIT MODIFIZIERTER TRENNBESCHICHTUNG
CAPSULES À COQUE DURE DOTÉES D'UN REVÊTEMENT À LIBÉRATION MODIFIÉE

(30) Priority: 25.05.2021 EP 21175685
(43) Date of publication of application: 10.04.2024
(73) Proprietor: Evonik Operations GmbH, 45128 Essen (DE)
(72) Inventor: BÄR, Hans, 64720 Michelstadt (DE); HOFMANN, Felix, 64285 Darmstadt (DE); SMITH, Steven, 63263 Neu-Isenburg (DE); NIEPOTH, Peter, 64823 Groß-Umstadt (DE); JABER, Rima, 60327 Frankfurt (DE); HÖLZER, Bettina, 64625 Bensheim (DE)
(74) Representative: Evonik Patent Association
(86) International application number: PCT/EP2022/063581
(87) International publication number: WO 2022/248334

(56) References cited:
- WO-A1-2020/229178
- WO-A2-2011/012369
- US-A1- 2011 033 530

## Description

### FIELD OF THE INVENTION

The invention refers to a process for preparing a polymer-coated hard shell capsule, wherein the hard shell capsule comprises a body and a cap, wherein in the closed state the cap overlaps the body either in a pre-locked state or in a final-locked state, wherein the hard shell capsule is provided in the pre-locked state and coated with a coating solution, suspension or dispersion comprising or consisting of
a) at least one polymer;
b) at least one glidant;
c) at least one emulsifier;
d) optionally at least one plasticizer;
e) optionally at least one biologically active ingredient; and
f) optionally at least one additive, different from a) to e);
to obtain a coated, preferably only at the outer surface, hard shell capsule in the pre-locked state, wherein the coating solution, suspension or dispersion has a surface tension of at most 38 mN/m. Furthermore, the invention refers to a polymer-coated hard shell capsule obtained from the process according to the invention and the use of the polymer-coated hard shell capsule for immediate, delayed or sustained release.

### BACKGROUND

Polymer-coated hard shell capsules in the field of pharmaceutical or nutraceutical products are well known. They are for examples disclosed in WO 2019/096833 A1, WO 2020/229178 A1, and WO 2020/229192 A1. However, in the applications the coating process was only performed in a lab scale set-up of less than 9,000 capsules.

WO 2020/229178 A1 example 7 discloses a process for preparing a coated hard shell capsule, wherein the hard shell capsule coated with a coating solution. The coating solution comprises at least one glidant and emulsifier.

WO 2011/012369 A1 and US 2011/033530 A1 disclose a coating agent for the gastric juice-resistant coating of capsule halves, which are made of a water-soluble or water-swellable polymer material. The coating agent is provided in the form of an aqueous dispersion or solution containing a polymer mixture of at least one (meth)acrylate copolymer that is gastric juice-resistant, as well as auxiliary materials that influence the viscosity of the dispersion and the elasticity of the dried polymer film. The aqueous dispersion or solution is characterized in that the solids content of the dispersion or solution is more than 25 percent by weight and the viscosity is 150 to 1,500 mPas, wherein a film generated by the dispersion or solution and dried has an ultimate elongation of at least 200 percent.

Even though the examples work well in those lab scale set-ups, the disclosed coatings are not suitable for industrial scale production methods. In particular, the inventors of the present invention surprisingly found that during the scale-up of the empty hard capsule coating process certain formulations as described in the prior art documents show increased bridging tendencies. Bridging tendencies means that the pre-locked coated empty capsules showed polymer bridges between the capsule body and capsule cap. This polymer bridges make them unsuitable for industrial production methods. Since for the downstream process like manual, semiautomated or automated capsule filling processes it is essential that the capsules can be opened and when opened that they are damage free.

In this regard, it has been surprisingly found by the inventors that the addition of at least one glidant and at least one emulsifier as well the use of a coating solution, suspension or dispersion having a surface tension of at most 38 mN/m can prevent polymer bridges in the final coatings prepared in pilot or manufacturing scale. Thus, the objective of the presently claimed invention is to provide coating solution, suspension or dispersion having a surface tension of at most 38 mN/m, which can prevent polymer bridge.

That is particularly unexpected, since surface active substances like emulsifiers typically decrease the glass transition temperature of polymers which is typically leading to increased sticking tendencies.

### SUMMARY OF THE INVENTION

In a first aspect the invention refers to a process for preparing a polymer-coated hard shell capsule, wherein the hard shell capsule comprises a body and a cap, wherein in the closed state the cap overlaps the body either in a pre-locked state or in a final-locked state, wherein the hard shell capsule is provided in the pre-locked state and coated with a coating solution, suspension or dispersion comprising or consisting of
a) at least one polymer;
b) at least one glidant;
c) at least one emulsifier;
d) optionally at least one plasticizer;
e) optionally at least one biologically active ingredient; and
f) optionally at least one additive, different from a) to e);
to obtain a coated, preferably only at the outer surface, hard shell capsule in the pre-locked state, wherein the coating solution, suspension or dispersion has a surface tension of at most 38 mN/m.

### DETAILED DESCRIPTION OF THE INVENTION

### HARD SHELL CAPSULES

Hard shell capsules for pharmaceutical or nutraceutical purposes are well known to a skilled person. A hard shell capsule is a two-piece encapsulation capsule comprising of the two capsule halves, called the body and the cap. The capsule body and cap material is usually made from a hard and sometimes brittle material. The hard shell capsule comprises a body and a cap. Body and cap are usually of a one end open cylindrical form with closed rounded hemispherical ends on the opposite end. The shape and size of the cap and body are such that the body can be pushed telescopically with its open end into the open end of the cap.

The body and the cap comprise a potential overlapping, matching area (overlap area) outside the body and inside the cap which partially overlap when the capsule is closed in the pre-locked state and totally overlap in the final-locked state. When the cap is partially slid over the overlapping matching area of the body the capsule is in the pre-locked state. When the cap is totally slid over the overlapping matching area of the body the capsule is in the final-locked state. The maintenance of the pre-locked state or of the final-locked state is usually supported by snap-in locking mechanisms of the body and the cap such as matching encircling notches or dimples, preferably elongated dimples.

Usually the body is longer than the cap. The outside overlapping area of the body can be covered by the cap in order to close or to lock the capsule. In the closed state the cap covers the outside overlap area of the body either in a pre-locked state or in a final-locked state. In the final-locked state the cap covers the outside overlap area of the body in total, in the pre-locked state the cap overlaps the outside overlapping area of the body only partially. The cap can be slid over the body to be fixed in usually one of two different positions in which the capsule is closed either in a pre-locked state or in a final-locked state.

Hard shell capsules are commercially available in different sizes. Hard shell capsules are usually delivered as empty containers with the body and cap already positioned in the pre-locked state and on demand as separate capsules halves, bodies and caps. The pre-locked hard shell capsules can be provided to a capsule-filling machine, which performs the opening, filling and closing of the capsule into the final-locked state. Usually hard shell capsules are filled with dry materials, for instance with powders or granules, or viscous liquids comprising a biologically active ingredient.

The cap and body are provided with closure means that are advantageous for the pre-locking (temporary) and/or final locking of the capsule. Therefore, elevated points can be provided on the inner wall of the cap and somewhat larger indented points are provided on the outer wall of the body, which are arranged so that when the capsule is closed the elevations fit into the indentations. Alternatively, the elevations can be formed on the outer wall of the body and the indentations on the inner wall of the cap. Arrangements in which the elevations or indentations arranged in a ring or spiral around the wall. Instead of the point-like configuration of the elevations and indentations, these may encircle the wall of the cap or body in an annular configuration, although advantageously recesses and openings are provided which enable an exchange of gases into and out of the capsule interior. One or more elevations can be provided in an annular arrangement around the inner wall of the cap and the outer wall of the body such that, in the final-locked position of the capsule, an elevation on the cap is located adjacent to an elevation on the body. Sometimes elevations are formed on the outside of the body close to the open end and indentations are formed in the cap close to the open end such that the elevations on the body latch into the indentations in the cap in the final-locked position of the capsule. The elevations can be such that the cap can be opened in the pre-locked state at any time without damage to the capsule or, alternatively, so that once it has been closed the capsule cannot be opened again without destroying it. Capsules with one or more such latching mechanisms (latches) (for example two encircling grooves) are preferred. More preferred are capsules with at least two such latching means which secure the two capsule parts to different degrees. In a part of this kind, a first latching (dimples or encircling notches) means can be formed close to the openings in the capsule cap and the capsule body and a second latching (encircling notches) can be shifted somewhat further towards the closed end of the capsule parts. The first latching means secure the two capsule parts less strongly than the second does. This variant has the advantage that after the production of the empty capsules the capsule cap and capsule body can initially be pre-locked joined together using the first latching mechanism. In order to fill the capsule the two capsule parts are then separated again. After filling, the two capsule parts are pushed together until the second set of latches firmly secures the capsule parts in a final-locked state.

Preferably, the body and the cap of the hard shell capsule are comprising each encircling notches and/or dimples in the area, where the cap can be slid over the body. Encircling notches of the body and dimples of the cap match to each other to provide a snap-in or snap into-place mechanism. The dimples can be circular or elongated (oval) in the longitudinal direction. Encircling notches of the body and encircling notches of the cap (closely matched rings) also match to each other to provide a snap-in or snap into-place mechanism. This allows the capsule to be closed by a snap-into-place mechanism either in a pre-locked state or in a final-locked state.

Preferably, matching encircling notches of the body and elongated dimples of the cap are used to fix the body and the cap to each other in the pre-locked state. Matching encircling notches of the body and the cap are preferably used to fix or lock the body and the cap to each other in the final-locked state.

The area, where the cap can be slid over the body can be called the overlapping area of the body and the cap or briefly the overlap area. If the cap overlaps the body only partially, maybe to 20 to 90 or 60 to 85 % of the overlap area, the hard shell capsule is only partially closed (pre-locked). Preferably, in the presence of a locking mechanism, like matching encircling notches and/or dimples in body and cap, the partially closed capsule can be called pre-locked. When the capsule is polymer-coated in the pre-locked state the coating will cover the completely outer surface including that part of the overlap area of the body and cap that is not overlapped by the cap in this pre-locked state. When the capsule is polymer-coated in the pre-locked state and then closed to the final-locked state the coating of that part of the overlap area of the body and cap that was not overlapped by the cap in the pre-locked state will then become covered by the cap. The presence of that part of the coating which is then enclosed in the final-locked state between the body and the cap is sufficient for the hard shell capsule to be tightly sealed.

If the cap overlaps the body the total overlapping area of the body, the hard shell capsule is finally closed or in the final-locked state. Preferably, in the presence of a locking mechanism, like matching encircling notches and/or dimples in body and cap, the finally closed capsule can be called final-locked.

Usually dimples are preferred for the fixing the body and the cap in the pre-locked state. As a non-binding rule the matching area of dimples is smaller than the matching area of encircling notches. Thus snapped-in dimples can be snapped-out again by applying less forces than those that would be necessary to snap-out a snapped-in fixation by matching encircling notches.

The dimples of the body and cap are located in the area, where the cap can be slid over the body match to each other in the pre-locked state by a snap in or snap into-place mechanism. There can be for example 2, 4, or preferably 6 notches or dimples located distributed circular around the cap.

Usually the dimples of the cap are and the encircling notches of the body in the area, where the cap can be slid over the body match to each other so that they that allow the capsule to be closed by a snap-into-place mechanism in the pre-locked state. In the pre-locked state, the hard shell capsule can be re-opened manually or by a machine without damaging, because the forces needed to open are comparatively low. Thus, the "pre-locked state" is sometimes designated also as "loosely capped".

Usually the encircling notches or matching locking rings of the body and the cap in the area, where the cap can be slid over the body match to each other so that they that allow the capsule to be closed by a snap-into-place mechanism in the final-locked state. In the final-locked state, the hard shell capsule cannot or can be only hardly be re-opened manually or by a machine without damaging, because the forces needed to open are comparatively high.

Usually dimples and the encircling notches are formed in the capsule body or capsule cap. When the capsule parts provided with these elevations and indentations are fitted into one another, ideally defined uniform gaps of from 10 microns to 150 microns, more particularly 20 microns to 100 microns, are formed along the contact surface between the capsule body and the capsule cap placed thereon.

Preferably, the body of the hard shell capsule comprises a tapered rim. The tapered rim prevent the rims of the body and the cap to collide and becoming damaged when the capsule is closed manually or by a machine.

In contrast to a hard shell capsule, a soft shell capsule is a welded one piece encapsulation capsule. A soft gel capsule is often made from blow molded soft gelling substances and is usually filled with liquids comprising a biologically active ingredient by injection. The invention is not concerned with welded soft shell one piece encapsulation capsules.

### SIZES OF HARD SHELL CAPSULES

A closed, final-locked hard shell capsule can have a total length in the range from about 5 to 40 mm. The diameter of the cap can be in the range from about 1.3 to 12 mm. The diameter of the body can be in the range from about 1.2 to 11 mm. The length of the cap can be in the range from about 4 to 20 mm and that of the body in the range from 8 to 30 mm. The fill volume can be between about from 0.004 to 2 ml. The difference between the pre-locked length and the final-locked length can be about 1 to 5 mm.

Capsules can be divided into standardized sizes for example from sizes 000 to 5. A closed capsule of size 000 has, for example, a total length of about 28 mm with an outer diameter of the cap of about 9.9 mm and an outer diameter of the body of about 9.5 mm. The length of the cap is about 14 mm, that of the body about 22 mm. The fill volume is about 1.4 ml.

A closed capsule of size 5 has, for example, a total length of about 10 mm and an outer diameter of the cap of about 4.8 mm and an outer diameter of the body of about 4.6 mm. The length of the cap is about 5.6 mm, that of the body about 9.4 mm. The fill volume is about 0.13 ml.

A size 0 capsule may show a length of about 23 to 24 mm in the pre-locked state and of about 20.5 to 21.5 mm in the final-locked state. Thus, the difference between the pre-locked length and the final-locked length can be about 2 to 3 mm.

### COATED HARD SHELL CAPSULE

The invention is concerned with a polymer-coated hard shell capsule, obtained by the process as described herein.

### Material of the body and the cap

The base material of the body and the cap can be selected from hydroxypropyl methyl cellulose, starch, gelatin, pullulan and a copolymer of C1- to C4-alkylester of (meth)acrylic acid and (meth)acrylic acid. Preferred are hard shell capsules where body and cap are comprising or consisting of HPMC or gelatin, most preferred is HPMC because of its good adhesion properties for the polymer coating.

### POLYMER OR POLYMER MIXTURE COMPRISED IN THE COATING LAYER

The at least one polymer comprised in the coating layer is preferably a film-forming polymer and can be selected from the group of anionic polymers, cationic polymers and neutral polymers or any mixture thereof.

The selection of generic or specific polymer features or embodiments as disclosed herein can be combined without restriction with any other generic or specific selection of material or numerical features or embodiments as disclosed herein, such as capsule materials, capsule sizes, coating thicknesses, biologically active ingredients and any other features or embodiments as disclosed.

The coating layer, which can be a single layer or can comprise or consist of two or more individual layers, can comprise in total 10 to 100, 20 to 95, 30 to 90 % by weight of one or more polymers, preferably (meth)acrylate copolymer(s).

The proportions of monomers mentioned for the respective polymers in general add up to 100% by weight.

### GLASS TRANSITION TEMPERATURE T_{GM}

The coating layer can comprise one or more polymers, preferably (meth)acrylate copolymer(s), with a glass transition temperature *T_{gm}* of 125 °C or less, preferably from -10 to 115 °C.

The coating layer can comprise one or more anionic cellulose(s), ethyl cellulose and/or one or more starches comprising at least 35 % by weight amylose with a glass transition temperature *T_{gm}* of 130 or less, preferably 127 °C or less, more preferably from 50 to 127 °C.

The glass transition temperature *T_{gm}* is determined by Differential Scanning Calorimetry (DSC) according to ISO 11357-2:2013-05. The determination is performed with a heating rate of 20 K/min. The glass transition temperature *T_{gm}* was determined by half step height method as described in section 10.1.2 of DIN EN ISO 11357-2.

### ANIONIC POLYMERS - ENTERIC COATING AND GASTRIC RESISTANCE

The described process is especially useful for providing tightly closed polymer-coated hard shell capsules for pharmaceutical or nutraceutical dosage forms with gastric resistance and an intended rapid release in the small intestine (enteric coating) or large intestine (colon targeting).

The at least one polymer comprised in the coating layer can be an anionic polymer selected from the groups of anionic (meth)acrylate copolymers, anionic polyvinyl polymers or copolymers and anionic celluloses.

The above-mentioned anionic polymers are also called "enteric polymers". In the coating layer such polymers are capable of providing enteric protection to the capsule. Enteric protection shall mean, when the capsule is in the final closed state and comprises a fill comprising a pharmaceutical or nutraceutical biologically active ingredient, less than 10 % of the comprised biologically active ingredient will be released after 120 min in 0.1 HCl, pH 1.2. Most preferred after 120 min in 0.1 HCl pH 1.2 and subsequent change to a buffered medium of pH 6.8 about 80 % or more of the comprised biologically active ingredient will be released after a total time of 165 min or 180 min. Colon targeting shall mean, when the capsule is in the final closed state and comprises a fill comprising a pharmaceutical or nutraceutical biologically active ingredient, less than 10 % of the comprised biologically active ingredient will be released after 120 min in 0.1 HCl, pH 1.2. Preferred after 120 min in 0.1 HCl pH 1.2 and subsequent change to a buffered medium of pH 6.8 about 80 % or more of the comprised biologically active ingredient will be released after a total time of 165 min. Most preferred after 120 min in 0.1 HCl pH 1.2 and 60 min at a subsequent intermediate change to a buffered medium of pH 6.5 or 6.8 and subsequent final change to a buffered medium of pH 7.2 or pH 7.4 about 80 % or more of the comprised biologically active ingredient will be released after a total time of 225 min or 240 min.

The dissolution test is performed according to the United States Pharmacopeia 43 (USP) chapter <711> utilizing USP Apparatus II with a paddle speed of 50 or 75 rpm. The test media temperature will be adjusted to 37 + 0.5°C. Samples will be taken at appropriate time points.

### ANIONIC (METH)ACRYLATE COPOLYMERS

Preferably the anionic (meth)acrylate copolymer comprises 25 to 95, preferably 40 to 95, in particular 60 to 40, % by weight free-radical polymerized C1- to C12-alkyl esters, preferably C1- to C4-alkyl esters of acrylic or of methacrylic acid and 75 to 5, preferably 60 to 5, in particular 40 to 60 % by weight (meth)acrylate monomers having an anionic group. The proportions mentioned in general add up to 100% by weight. However, it is also possible in addition, without this leading to an impairment or alteration of the essential properties, for small amounts in the region of 0 to 10, for example 1 to 5, % by weight of further monomers capable of vinylic copolymerization, such as, for example, hydroxyethyl methacrylate or hydroxy-ethyl acrylate, to be present. It is preferred that no further monomers capable of vinylic copolymerization are present.

C1- to C4-alkyl esters of acrylic or methacrylic acid are in particular methyl methacrylate, ethyl methacrylate, butyl methacrylate, methyl acrylate, ethyl acrylate and butyl acrylate.

A (meth)acrylate monomer having an anionic group is, for example, acrylic acid, with preference for methacrylic acid.

Suitable anionic (meth)acrylate copolymers are those polymerized from of 40 to 60% by weight methacrylic acid and 60 to 40% by weight methyl methacrylate or 60 to 40% by weight ethyl acrylate (EUDRAGIT^{®} L or EUDRAGIT^{®} L 100 55 types).

EUDRAGIT^{®} L is a copolymer polymerized from 50% by weight methyl methacrylate and 50% by weight methacrylic acid. The pH of the start of the specific active ingredient release in intestinal juice or simulated intestinal fluid can be stated to be at about pH value 6.0.

EUDRAGIT^{®} L 100-55 is a copolymer polymerized from 50% by weight ethyl acrylate and 50% by weight methacrylic acid. EUDRAGIT^{®} L 30 D-55 is a dispersion comprising 30% by weight EUDRAGIT^{®} L 100-55. The pH of the start of the specific active ingredient release in intestinal juice or simulated intestinal fluid can be stated to be at about pH value 5.5.

Likewise, suitable are anionic (meth)acrylate copolymers polymerized from 20 to 40% by weight methacrylic acid and 80 to 60% by weight methyl methacrylate (EUDRAGIT^{®} S type). The pH value of the start of the specific active ingredient release in intestinal juice or simulated intestinal fluid can be stated to be at about pH value 7.0.

Suitable (meth)acrylate copolymers are polymerized from 10 to 30% by weight methyl methacrylate, 50 to 70% by weight methyl acrylate and 5 to 15% by weight methacrylic acid (EUDRAGIT^{®} FS type). The pH at the start of the specific active ingredient release in intestinal juice or simulated intestinal fluid can be stated to be at about pH value 7.0.

EUDRAGIT^{®} FS is a copolymer polymerized from 25% by weight methyl methacrylate, 65% by weight methyl acrylate and 10% by weight methacrylic acid. EUDRAGIT^{®} FS 30 D is a dispersion comprising 30% by weight EUDRAGIT^{®} FS.

Suitable is a copolymer composed of
20 to 34% by weight methacrylic acid and/or acrylic acid,
20 to 69% by weight methyl acrylate and
0 to 40% by weight ethyl acrylate and/or where appropriate
0 to 10% by weight further monomers capable of vinylic copolymerization,
with the proviso that the glass transition temperature of the copolymer according to ISO 11357-2:2013-05, subsection 3.3.3, is not more than 60°C. This (meth)acrylate copolymer is particularly suitable, because of its good elongation at break properties, for compressing pellets to tablets.

Suitable is a copolymer polymerized from
20 to 33% by weight methacrylic acid and/or acrylic acid,
5 to 30% by weight methyl acrylate and
20 to 40% by weight ethyl acrylate and
more than 10 to 30% by weight butyl methacrylate and where appropriate
0 to 10% by weight further monomers capable of vinylic copolymerization,
where the proportions of the monomers add up to 100% by weight,
with the proviso that the glass transition temperature of the copolymer according to ISO 11357-2:2013-05, subsection 3.3.3 (midpoint temperature Tmg), is 55 to 70°C.

The copolymer preferably consists of 90, 95 or 99 to 100% by weight of the monomers methacrylic acid, methyl acrylate, ethyl acrylate and butyl methacrylate in the ranges of amounts indicated above. However, it is possible, without this necessarily leading to an impairment of the essential properties, for small amounts in the range from 0 to 10, e.g. 1 to 5% by weight of further monomers capable of vinylic copolymerization additionally to be present, such as, for example, methyl methacrylate, butyl acrylate, hydroxyethyl methacrylate, vinylpyrrolidone, vinyl-malonic acid, styrene, vinyl alcohol, vinyl acetate and/or derivatives thereof.

Further suitable anionic (meth)acrylate copolymers can be so called core/shell polymers as described in WO 2012/171575 A2 or WO 2012/171576 A1. A suitable Core Shell polymer is a copolymer from a two stage emulsion polymerization process with a core of 75 % by weight comprising polymerized units of 30% by weight of ethyl acrylate and 70% by weight of methyl methacrylate and a shell of polymerized units comprising 25 % by weight of polymerized from 50% by weight ethyl acrylate and 50% by weight methacrylic acid.

A suitable Core-Shell polymer can be a copolymer from a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid.

### ANIONIC CELLULOSES

Anionic celluloses can be selected from carboxymethyl ethyl cellulose and its salts, cellulose acetate phthalate (CAP), cellulose acetate succinate (CAS), cellulose acetate trimellitate (CAT), hydroxypropyl methyl cellulose phthalate (HPMCP, HP50, HP55), hydroxypropyl methyl cellulose acetate succinate (HPMCAS-LF, -MF, -HF).

The hard shell capsule is coated with a coating layer that covers the hard shell capsule in the pre-locked state. The coating layer can comprises one or more anionic cellulose(s), ethyl cellulose and/or one or more starches comprising at least 35 % by weight amylose, preferably with a glass transition temperature *T_{gm}* of 130 °C or less (determined by Differential Scanning Calorimetry (DSC) according to ISO 11357-2:2013-05), wherein the coating layer is preferably present in an amount of about 1 to 5.8, more preferably 2 to 5 mg/cm².

The coating layer, which can be a single layer or may comprise or consist of two or more individual layers, may comprise in total 10 to 100, 20 to 95, 30 to 90 % by weight of comprising one or more anionic cellulose(s), ethyl cellulose and/or one or more starches comprising at least 35 % by weight amylose.

The glass transition temperature *T_{gm}* of hydroxypropyl methyl cellulose phthalate is about 132 to 138 °C (type HP-55 about 133 °C, type HP-50 about 137 °C).

The glass transition temperature *T_{gm} of* hydroxypropyl methyl cellulose acetate succinate (HPMCAS) is about 120 °C (AquaSolve^{™} L HPMCAS 119°C, AquaSolve^{™} M HPMCAS 120°C, AquaSolve^{™} H HPMCAS 122°C).

### ETHYL CELLULOSE

Ethyl cellulose is a derivative of cellulose in which some of the hydroxyl groups of the repeating glucose units are converted into ethyl ether groups. Ethyl cellulose can be used as a delayed release coating material for the capsules as disclosed. The glass transition temperature *T_{gm}* of ethyl cellulose can be in the range of about 128 to 130 °C (Hui Ling Lai et al. Int.J.Pharmaceuticals 386 (2010) 178-184).

### STARCHES COMPRISING AT LEAST 35 % BY WEIGHT AMYLOSE

Starches comprising at least 35 % by weight amylose are commercially available as starch from corn or maize origin.

Starches comprising at least 35 % by weight amylose are known for example from EP 1296658 B1. This type of chemically modified (acetylated) starch with a high content in amylose is obtained through a pre-gelation process. These starches show a high mechanical resistance for the production of capsules and coatings for solid formulations used in various application in the fields of pharmaceuticals or nutraceuticals.

The glass transition temperature *T_{gm}* of starches comprising at least 35 % by weight amylose can be in the range of about 52 to 60 °C (Peng Liu et al., J.Cereal Science (2010) 388-391).

### ANIONIC VINYL COPOLYMERS

Anionic vinyl copolymers can be selected from unsaturated carboxylic acids other than acrylic acid or methacrylic acid as exemplified by polyvinylacetatephthalate or a copolymer of vinylacetate and crotonic acid (preferably at a ratio of 9:1).

### CATIONIC POLYMERS - MOISTURE PROTECTION

The described process is especially useful for providing polymer-coated hard shell capsules and pharmaceutical or nutraceutical dosage forms based on these kind of capsules with improved moisture protection properties, e.g. with decreased moisture up-take during storage. For this purpose a coating with a cationic polymer, preferably with cationic (meth)acrylate copolymer is suggested.

A suitable cationic (meth)acrylate copolymer comprised in the coating layer can be polymerized from monomers comprising C1- to C4-alkyl esters of acrylic or of methacrylic acid and an alkyl ester of acrylic or of methacrylic acid with a tertiary or a quaternary ammonium group in the alkyl group. The cationic, water-soluble (meth)acrylate copolymer can be polymerized partly or fully of alkyl from acrylates and/or alkyl methacrylates having a tertiary amino group in the alkyl radical. A coating comprising these kind of polymers may have the advantage of providing moisture protection to the hard shell capsule. Moisture protection shall be understood a reduced uptake of moisture or water during storage of the readily filled and final-locked capsules.

A suitable cationic (meth)acrylate copolymer can be polymerized from 30 to 80% by weight of C1-to C4-alkyl esters of acrylic or of methacrylic acid, and 70 to 20% by weight of alkyl(meth)acrylate monomers having a tertiary amino group in the alkyl radical.

The preferred cationic (meth)acrylate copolymer can be polymerized from 20 - 30% by weight of methyl methacrylate, 20 - 30% by weight of butyl methacrylate and 60 - 40% by weight of dimethylaminoethyl methacrylate (EUDRAGIT^{®} E type polymer).

A specifically suitable commercial (meth)acrylate copolymer with tertiary amino groups is polymerized from 25% by weight of methyl methacrylate, 25% by weight of butyl methacrylate and 50% by weight of dimethylaminoethyl methacrylate (EUDRAGIT^{®} E 100 or EUDRAGIT^{®} E PO (powder form)). EUDRAGIT^{®} E 100 and EUDRAGIT^{®} E PO are water-soluble below approx. pH value 5.0 and are thus also gastric juice-soluble.

A suitable (meth)acrylate copolymer can be composed of 85 to 98% by weight of free-radical polymerized C1 to C4 alkyl esters of acrylic or methacrylic acid and 15 to 2% by weight of (meth)acrylate monomers with a quaternary amino group in the alkyl radical.

Preferred C1 to C4 alkyl esters of acrylic or methacrylic acid are methyl acrylate, ethyl acrylate, butyl acrylate, butyl methacrylate and methyl methacrylate.

Further suitable cationic (meth)acrylate polymers may contain polymerized monomer units of 2-trimethylammonium-ethyl methacrylate chloride or trimethylammonium-propyl methacrylate chloride.

An appropriate copolymer can be polymerized from 50 to 70% by weight of methyl methacrylate, 20 to 40% by weight of ethyl acrylate and 7 to 2% by weight of 2-trimethylammoniumethyl methacrylate chloride.

A specifically suitable copolymer is polymerized from 65% by weight of methyl methacrylate, 30% by weight of ethyl acrylate and 5% by weight of 2-trimethylammoniumethyl methacrylate chloride (EUDRAGIT^{®} RS).

A further suitable (meth)acrylate copolymer can be polymerized from 85 to less than 93% by weight of C1 to C4 alkyl esters of acrylic or methacrylic acid and more than 7 to 15% by weight of (meth)acrylate monomers with a quaternary amino group in the alkyl radical. Such (meth)acrylate monomers are commercially available and have long been used for release-slowing coatings.

A specifically suitable copolymer is polymerized from 60% by weight of methyl methacrylate, 30% by weight of ethyl acrylate and 10% by weight of 2-trimethylammoniumethyl methacrylate chloride (EUDRAGIT^{®} RL).

### NEUTRAL POLYMERS

Neutral polymers are defined as polymers which are polymerized from neutral monomers and less than 5, preferably less than 2 % by weight or most preferred no monomers with ionic groups.

Suitable neutral polymers for the coating of the hard shell capsule are methacrylate copolymers, preferably copolymers of ethyl acrylate and methyl methacrylate like EUDRAGIT^{®} NE or EUDRAGIT^{®} NM, neutral celluloses, such as methyl-, ethyl- or propyl ethers of cellulose, for instance hydroxypropyl cellulose, polyvinyl pyrrolidone, polyvinyl acetate or polyvinyl alcohol.

Neutral methacrylate copolymers are often useful in mixture with anionic (meth)acrylate copolymers.

Neutral methacrylate copolymers are polymerized from at least to an extent of more than 95% by weight, in particular to an extent of at least 98% by weight, preferably to an extent of at least 99% by weight, in particular to an extent of at least 99% by weight, more preferably to an extent of 100% by weight, of (meth)acrylate monomers with neutral radicals, especially C1- to C4-alkyl radicals.

Suitable (meth)acrylate monomers with neutral radicals are, for example, methyl methacrylate, ethyl methacrylate, butyl methacrylate, methyl acrylate, ethyl acrylate, butyl acrylate. Preference is given to methyl methacrylate, ethyl acrylate and methyl acrylate.

Methacrylate monomers with anionic radicals, for example acrylic acid and/or methacrylic acid, can be present in small amounts of less than 5% by weight, preferably not more than 2% by weight, more preferably not more than 1 or 0.05 to 1% by weight.

Suitable examples are neutral or virtually neutral (meth)acrylate copolymers polymerized from 20 to 40% by weight of ethyl acrylate, 60 to 80% by weight of methyl methacrylate and 0 to less than 5% by weight, preferably 0 to 2 or 0.05 to 1% by weight of methacrylic acid or acrylic acid.

Suitable examples are neutral or virtually neutral (meth)acrylate copolymers polymerized from 20 to 40% methyl methacrylate by weight of, 60 to 80% by weight of ethyl acrylate and 0 to less than 5% by weight, preferably 0 to 2 or 0.05 to 1% by weight of methacrylic acid or acrylic acid. (EUDRAGIT^{®} NE or EUDRAGIT^{®} NM type).

EUDRAGIT^{®} NE and EUDRAGIT^{®} NM are copolymers comprising free-radically polymerized units of 28 to 32% by weight of methyl methacrylate and 68 to 72% by weight of ethyl acrylate.

Preference is given to neutral or essentially neutral methyl acrylate copolymers which, according to WO 01/68767 A1, have been prepared as dispersions using 1 - 10% by weight of a non-ionic emulsifier having an HLB value of 15.2 to 17.3. The latter offer the advantage that there is no phase separation with formation of crystal structures by the emulsifier (EUDRAGIT^{®} NM type).

According to EP 1 571 164 A2, corresponding, virtually neutral (meth)acrylate copolymers with small proportions of 0.05 to 1% by weight of monoolefinically unsaturated C3-C8-carboxylic acids can, however, also be prepared by emulsion polymerization in the presence of comparatively small amounts of anionic emulsifiers, for example 0.001 to 1% by weight.

### NATURAL POLYMERS

Especially for nutraceutical dosage forms so called "natural polymer" coatings are preferred by many customers. Natural polymers are based on a source from nature, plants, microorganisms or animals, but sometimes further chemically processed. Natural polymers for coatings can be selected from polymers such as starch, alginates or salts of alginates, preferably sodium alginate, pectin, shellac, zein, carboxymethyl-zein, modified starch, for instance EUDRAGUARD^{®} Natural, marine sponge collagen, chitosan, gellan gum. Suitable polymer mixtures may comprise:
Ethyl cellulose and pectin, modified starch (EUDRAGUARD^{®} Natural) and alginate and/or pectin, shellac and alginate and/or pectin, shellac and inulin, whey protein and gums (such as guar gum or tragacanth gum), zein, sodium alginate and chitosan.

### Glidants

Glidants usually have lipophilic properties. They prevent agglomeration of cores during film formation of the film forming polymers.

The at least one glidant is preferably selected from silica, for example commercially available under the tradenames RXCIPIENTS^{®} GL100 or RXCIPIENTS^{®} GL200, ground silica, fumed silica, kaolin calcium silicate, magnesium silicate, colloidal silicone dioxide, talc, stearate salts like calcium stearate, magnesium stearate, zinc stearate, sodium stearyl fumarate, starch, stearic acid, preferably talc, magnesium stearate, colloidal silicon dioxide and glycerol monostearate or mixtures thereof, more preferred glycerol monostearate and talc or mixtures thereof.

Standard proportions for use of glidants in the inventive coating range between 0.5 and 100 % by weight, preferably 3 to 75 % by weight, more preferably 5 to 50 % by weight, most preferably 5 to 30 % by weight, relative to the total weight of the at least one polymer.

### Emulsifiers

In general, all known emulsifiers are suitable. Preferred are non-ionic emulsifier, in particular emulsifier having an HLB > 10. The HBL Value can be determined according to Griffin, William C. (1954), "Calculation of HLB Values of Non-Ionic Surfactants" (PDF), Journal of the Society of Cosmetic Chemists, 5 (4): 249-56.

The at least one emulsifier is preferably selected from polyglycosides, alcohols, sugar and sugar derivatives, polyethers, amines, polyethylene derivatives, alkyl sulfates (e.g. sodium dodecyl sulfate), alkyl ether sulfates, dioctyl sodium sulfosuccinate, polysorbates (e.g. polyoxyethylene (20) sorbitan monooleate), nonylphenol ethoxylates (nonoxynol-9) and mixtures thereof.

The at least one emulsifier is preferably selected from alkyl polyglycosides, decyl glucoside, decyl polyglucose, lauryl glucoside, octyl glucoside, N-octyl beta-D-thioglucopyranoside, cetostearyl alcohol, cetyl alcohol, stearyl alcohol, polyoxyethylene cetostearyl alcohol, cetylstearyl alcohol, oleyl alcohol, polyglyceryl-6-dioleate, glyceryl stearate citrate, polyglyceryl-3 caprate, polyglyceryl-3 diisostearate, glyceryl isostearate, polyglyceryl-4-isostearate, glyceryl monolinoleate, dicaprylyl carbonate, alcohol polyglycol ether, polyethylenglycolether of cetearylalcohols (n=20), polyethylene glycol-6 stearate, glycol stearate, polyethylene glycol-32 stearate, polyethylene glycol-20 stearate, fatty alcohol polyglycol ether, polyethylene glycol-4 laurate, polyethylene glycol isocetyl ether (n=20), polyethyleneglycol-32 (Mw 1500 g/mol) mono- and diesters of lauric acid (C12), nonaethylene glycol, polyethylene glycol nonylphenyl ether, octaethylene glycol monododecyl ether, pentaethylene glycol monododecyl ether, polyethylene glycol macrocetyl ether, polyethylene glycol esters of palmitic (C16) or stearic (C18) or caprylic acids, polyoxyethylene fatty ether derived from stearyl alcohols like BRIJ S2, polyoxyethylene oxypropylene stearate, macrogol stearyl ether (20), diethylaminoethyl stearate, polyethylene glycol stearate, sucrose distearate, sucrose tristearate, sorbitan monostearate, sorbitan tristearate, mannide monooleate, octaglycerol monooleate, sorbitan dioleate, polyricinoleate, polysorbate like polysorbate 20 and Polyoxyethylene (20) sorbitan monooleate (polysorbate 80), sorbitan, sorbitan monolaurate, sucrose cocoate, glycereth-2 cocoate, ethylhexyl cocoate, polypropylene glycol -3 benzyl ether myristate, sodium myristate, gold sodium thiomalate, polyethylene glycol 8 laurate, polyethylene-4 dilaurate, from α-Hexadecyl-ω-hydroxypoly(oxyethylene), cocamide diethanolamine, N-(2-hydroxyethyl)dodecanamide, octylphenoxypolyethoxyethanol, maltoside, 2,3-Dihydroxypropyl dodecanoate, 3-[(3R,6R,9R,12R,15S,22S,25S,30aS)-6,9,15,22-Tetrakis(2-amino-2-oxoethyl)-3-(4-hydroxybenzyl)-12-(hydroxymethyl)-18-(11-methyltridecyl)-1,4,7,10,13,16,20,23,26-nonaoxotriacontahydropyrrolo[1,2-g][1,4,7,10,13,16,19,22,25]nonaazacyclooctacosin-25-yl]propenamide, 2-{2-[2-(2-{2-[2-(2-{2-[2-(4-nonylphenoxy)ethoxy]ethoxy}ethoxy)ethoxy]ethoxy}ethoxy)ethoxy]ethoxy}ethanol, oxypolyethoxydodecane, poloxamers like poloxamer 188 (Pluronic F-68) and poloxamer 407, propylene glycol monocaprylate, type I (Capryol PGMC), polyethoxylated tallow amine, polyglycerol, polyoxyl 40 hydrogenated castor oil, surfactin, 2-[4-(2,4,4-trimethylpentan-2-yl)phenoxy]ethanol, carbomer, sodium carbomer, carboxymethylcellulose calcium, carrageenan, cholesterol, deoxycholic acid, phospholipids like egg phospholipids, gellan gum, lanolin, capric acid, waxes like Polawax NF, Polawax A31 or Ceral PW, ester gum, dea-cetyl phosphate, soya lecithin, sphingomyelins, sodium phosphate, sodium lauroyl lactylate, lanolin, Oxirane methyl-polymer with oxirane monobutyl ether, 1,2-dierucoylphosphatidylcholine, dimethicone end-blocked with an average of 14 moles of propylene oxide, laurylmethicone copolyol, lauroglycol 90, white mineral oil like Amphocerine KS, dispersion of acrylamide/sodium acryloyldimethyl taurate copolymer in isohexadecane, and sodium polyacrylate or mixtures thereof. Preferred are macrogol stearyl ether (20) and polysorbate 80.

### COATING LAYER

The hard shell capsule is coated with a coating layer comprising the at least one polymer, at least one glidant, at least one emulsifier and optionally at least one plasticizer, optionally at least one biologically active ingredient and optionally at least one additive, different from the beforementioned components.

The coating layer may comprise 10 % or more, 20 % or more, 30 % or more, 40 % or more, 50 % or more, 60 % or more, 70 % or more, 80 % or more, 90 % or more by weight or 95 % or more by weight of the at least one polymer. The coating layer may comprise 10 - 100, 10 - 90, 12 - 80, 15 - 80, 18 - 80, 20 - 80 or 40 to 80 % by weight of the at least one polymer.

### AMOUNT AND THICKNESS OF THE COATING LAYER

For hard shell capsules, the amount of the coating layer should not be too high. If the amount of coating layer applied is too high this may result in difficulties to process the polymer-coated pre-locked hard shell capsules subsequently in a capsule-filling machine. If the amount of coating layer is less than 8 mg/cm², for instance 1 to 8 mg/cm² or 1 to 7 mg/cm² or 1 to 7 mg/cm² or 1 to 6 mg/cm² or 1 to 5 mg/cm² or 1 to 4 mg/cm² usually no problem with standard capsule-filling machines without modification will occur. In the range from 4 and up to about 8 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 1 to about 8 mg/cm².

For a hard shell capsule of size #0, the amount of the coating layer should not be too high. If the amount of coating layer applied is too high this may result in difficulties to process the polymer-coated pre-locked hard shell capsules subsequently in a capsule-filling machine. If the amount of coating layer is less than 5 mg/cm², for instance 1 to 4 mg/cm² usually no problem with standard capsule-filling machines without modification will occur. In the range from 4 and up to about 8 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 1 to about 8 mg/cm².

For a hard shell capsule of size #1, the amount of the coating layer should not be too high. If the amount of coating layer applied is too high this may result in difficulties to process the polymer-coated pre-locked hard shell capsules subsequently in a capsule-filling machine. If the amount of coating layer is less than 4 mg/cm², for instance 1 to 3.5 mg/cm² usually no problem with standard capsule-filling machines without modification will occur. In the range from 3.5 and up to about 8 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 1 to about 8 mg/cm².

For a hard shell capsule of size #3, the amount of the coating layer should not be too high. If the amount of coating layer applied is too high this may result in difficulties to process the polymer-coated pre-locked hard shell capsules subsequently in a capsule-filling machine. If the amount of coating layer is less than 3 mg/cm², for instance 1 to 2.5 mg/cm² usually no problem with standard capsule-filling machines without modification will occur. In the range from 2.5 and up to about 6 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 1 to about 6 mg/cm².

Above 8 mg/cm² and up to about 20 mg/cm² careful manual opening of the polymer-coated hard shell capsule, filling and closing to the pre-locked state may still be possible without causing damage to the polymer coating. If the coating layer is thicker than the gap between the uncoated body and the cap, the coated pre-locked capsules cannot be closed without damaging the applied coating as the cap can hardly slide over the body to the final-locked state anymore. The upper limit for manual closing of coated pre-locked hard shell capsules to the final-locked state without causing damage can be up to an amount of the coating layer of about 20 mg/cm². Above 20 mg/cm² even a very accurate and careful manual closing of the capsule can be no more possible without causing damage.

If the amount of coating layer applied is too high there will be also an assembly of too much coating layer at the rim of the cap where the gap between body and cap is in the pre-locked state. This may result after drying in fissures of the coating layer when the coated pre-locked hard shell capsule is opened manually or in a machine. The fissures may result in a later leakage of the capsule. Finally, a too thick coating may result in difficulties or make it impossible to close the opened coated hard shell capsule to the final-locked state since the coating layer is thicker than the gap in the overlapping area between the body and the cap.

As a rough rule the coating layer on the hard shell capsule can be applied in an amount (= a total weight gain) of 0.7 to 20, 1.0 - 18, 2 to 10, 4 to 8, 1.0 to 8, 1.5 to 5.5, 1.5 to 4mg/cm².

As a rough rule the coating layer on the hard shell capsule may have an average thickness of about 5 to 100, 10 to 50, 15 to 75 µm.

As a rough rule the coating layer on the hard shell capsule can be applied in an amount of 5 to 50, preferably 8 - 40 % dry weight in relation to the weight of the pre-locked capsule.

With this guidance a skilled person will be able to adjust the amounts of the coating layer in a range between too low and too high.

### BIOLOGICALLY ACTIVE INGREDIENT

The biologically active ingredient is preferably a pharmaceutical active ingredient and/or a nutraceutical active ingredient and/or a cosmetically active ingredient. Even though it is possible that certain biologically active ingredients are contained in the respective coating layers, it is preferred that the biologically active ingredient is contained in the fill-in. In particular, if the biologically active ingredient is a liposome, lipid nanoparticle or nucleic acid, the biologically active ingredient is only contained in the fill-in.

### Pharmaceutical or nutraceutical active ingredients

The invention is preferably useful for immediate, delayed release or sustained release formulated pharmaceutical or nutraceutical dosage forms with a fill-in of pharmaceutical or nutraceutical active ingredients.

Suitable therapeutic and chemical classes of pharmaceutical active ingredients which members can be used as fill-in for the described polymer-coated hard shell capsules are for instance: analgesics, antibiotics or anti-infectives, antibodies, antiepileptics, antigens from plants, antirheumatics, benzimidazole derivatives, beta-blocker, cardiovascular drugs, chemotherapeutics, CNS drugs, digitalis glycosides, gastrointestinal drugs, e.g. proton pump inhibitors, enzymes, hormones, liquid or solid natural extracts, oligonucleotides, peptide, hormones, proteins, therapeutic bacteria, peptides, proteins (metal)salt i.e. aspartates, chlorides, urology drugs, lipid nanoparticles, liposomes, polymeric nanoparticles, vaccines.

In a preferred embodiment the pharmaceutically active ingredient is a nucleic acid, more preferably a nucleic acid agent can be DNA, RNA, or combinations thereof. In some embodiments, a nucleic acid agent can be an oligonucleotide and/or polynucleotide. In some embodiments, a nucleic acid agent may be an oligonucleotide and/or modified oligonucleotide (including, but not limited to, modifications through phosphorylation); an antisense oligonucleotide and/or modified antisense oligonucleotide (including, but not limited to, modifications through phosphorylation). In some embodiments, a nucleic acid agent can comprise cDNA and/or genomic DNA. In some embodiments, a nucleic acid agent can comprise non-human DNA and/or RNA (e.g., viral, bacterial, or fungal nucleic acid sequences). In some embodiments, a nucleic acid agent can be a plasmid, cosmid, gene fragment, artificial and/or natural chromosome (e.g., a yeast artificial chromosome), and/or a part thereof. In some embodiments, a nucleic acid agent can be a functional RNA (e.g., a mRNA, a tRNA, an rRNA and/or a ribozyme). In some embodiments, a nucleic acid agent can be an RNAi-inducing agent, small interfering RNA (siRNA), short hairpin RNA (shRNA), and/or microRNA (miRNA). In some embodiments, a nucleic acid agent can be a peptide nucleic acid (PNA). In some embodiments, a nucleic acid agent can be a polynucleotide comprising synthetic analogues of nucleic acids, which may be modified or unmodified. In some embodiments, a nucleic acid agent can comprise various structural forms of DNA including single-stranded DNA, double-stranded DNA, supercoiled DNA and/or triple -helical DNA; Z-DNA; and/or combinations thereof. Further suitable nucleic acids are for example disclosed in WO 2012103035 A1.

Further examples of drugs that can be used as fill-in for the described polymer-coated hard shell capsules are for instance acamprosat, aescin, amylase, acetylsalicylic acid, adrenalin, 5-amino salicylic acid, aureomycin, bacitracin, balsalazine, beta carotene, bicalutamid, bisacodyl, bromelain, bromelain, budesonide, calcitonin, carbamacipine, carboplatin, cephalosporins, cetrorelix, clarithromycin, chloromycetin, cimetidine, cisapride, cladribine, clorazepate, cromalyn, 1-deaminocysteine-8-D-arginine-vasopressin, deramciclane, detirelix, dexlansoprazole, diclofenac, didanosine, digitoxin and other digitalis glycosides, dihydrostreptomycin, dimethicone, divalproex, drospirenone, duloxetine, enzymes, erythromycin, esomeprazole, estrogens, etoposide, famotidine, fluorides, garlic oil, glucagon, granulocyte colony stimulating factor (G-CSF), heparin, hydrocortisone, human growth hormon (hGH), ibuprofen, ilaprazole, insulin, Interferon, Interleukin, Intron A, ketoprofen, lansoprazole, leuprolidacetat lipase, lipoic acid, lithium, kinin, memantine, mesalazine, methenamine, milameline, minerals, minoprazole, naproxen, natamycin, nitrofurantion, novobiocin, olsalazine, omeprazole, orothates, pancreatin, pantoprazole, parathyroid hormone, paroxetine, penicillin, perprazol, pindolol, polymyxin, potassium, pravastatin, prednisone, preglumetacin progabide, pro-somatostatin, protease, quinapril, rabeprazole, ranitidine, ranolazine, reboxetine, rutosid, somatostatin streptomycin, subtilin, sulfasalazine, sulphanilamide, tamsulosin, tenatoprazole, thrypsine, valproic acid, vasopressin, vitamins, zinc, including their salts, derivatives, polymorphs, isomorphs, or any kinds of mixtures or combinations thereof.

It is evident to a skilled person that there is a broad overlap between the terms pharmaceutical and nutraceutical active ingredients, excipients and compositions respectively a pharmaceutical or a nutraceutical dosage form. Many substances listed as nutraceuticals may also be used as pharmaceutical active ingredients. Depending on the specific application and local authority legislation and classification, the same substance can be listed as a pharmaceutical or a nutraceutical active ingredient respectively a pharmaceutical or a nutraceutical composition or even both.

Nutraceuticals are well known to the skilled person. Nutraceuticals are often defined as extracts of foods claimed to have medical effects on human health. Thus, nutraceutical active ingredients may display pharmaceutical activities as well: Examples for nutraceutical active ingredients can be resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Thus, it is clear that many substances listed as nutraceuticals may also be used as pharmaceutical active ingredients.

Typical nutraceuticals or nutraceutical active ingredients that can be used as fill-in for the described polymer-coated hard shell capsules may also include probiotics and prebiotics. Probiotics are living microorganisms believed to support human or animal health when consumed. Prebiotics are nutraceuticals or nutraceutical active ingredients that induce or promote the growth or activity of beneficial microorganisms in the human or animal intestine.

Examples for nutraceuticals are resveratrol from grape products, omega-3-fatty acids or pro-anthocyanines from blueberries as antioxidants, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals or nutriceuticals are used as synonyms for nutraceuticals.

Preferred biologically active ingredients are metoprolol, mesalamine and omeprazole.

### ADDITIVES

Additives according to the present invention are preferably excipients, which are well known to a skilled person and often formulated along with the biologically active ingredient contained in the coated hard shell capsule and/or with the polymer coating of the hard shell capsule as disclosed and claimed herein. All excipients used must be toxicologically safe and be used in pharmaceuticals or nutraceuticals without risk for patients or consumers.

The dosage form may comprise excipients, preferably pharmaceutical or nutraceutical acceptable excipients, selected from the group of antioxidants, brighteners, binding agents, flavouring agents, flow aids, fragrances, penetration-promoting agents, pigments, plasticizers, pore-forming agents or stabilizers or combinations thereof. The pharmaceutically or nutraceutically acceptable excipients can be comprised in the core and/or in the coating layer comprising the polymer as disclosed. A pharmaceutical or nutraceutical acceptable excipient is an excipient, which is allowed to be used for the application in the pharmaceutical or nutraceutical field.

The coating layer may comprise up to 90, up to 80, up to 70, up to 50, up to 60, up to 50, up to 40, up to 30, up to 20, up to 10, up to 5 % up to 3 %, up to 1 % by weight or not any (0 %) additives at all, respectively pharmaceutically or nutraceutically acceptable excipients.

### Plasticizers

The polymer coating of the hard shell capsule may comprises one or more plasticizers. Plasticizers achieve through physical interaction with a polymer a reduction in the glass transition temperature and promote film formation, depending on the added amount. Suitable substances usually have a molecular weight of between 100 and 20,000 g/mol and comprise one or more hydrophilic groups in the molecule, e.g. hydroxyl, ester or amino groups.

Examples of suitable plasticizers are alkyl citrates, alkyl phthalates, alkyl sebacates, diethyl sebacate, dibutyl sebacate, polyethylene glycols, and polypropylene glycols. Preferred plasticizers are triethyl citrate (TEC), acetyl triethyl citrate (ATEC), diethyl sebacate, dibutyl sebacate (DBS), polyethylene glycols, and polypropylene glycols or mixtures thereof.

Addition of the plasticizers to the formulation can be carried out in a known manner, directly, in aqueous solution or after thermal pre-treatment of the mixture. It is also possible to employ mixtures of plasticizers. The polymer coating of the hard shell capsule may comprise one or more plasticizers, preferably up to 60, up to 30, up to 25, up to 20, up to 15, up to 10, up to 5, less than 5% by weight, calculated on the at least one polymer, of a plasticizer or any (0 %) plasticizer at all can be comprised.

### Fillers

Standard fillers are usually added to the inventive formulation during processing to coating and binding agents. The quantities introduced and the use of standard fillers in pharmaceutical coatings or over layers is familiar to those skilled in the art. Examples of standard fillers are release agents, pigments, stabilizers, antioxidants, pore-forming agents, penetration-promoting agents, brighteners, fragrances or flavoring agents. They are used as processing adjuvants and are intended to ensure a reliable and reproducible preparation process as well as good long-term storage stability, or they achieve additional advantageous properties in the pharmaceutical form. They are added to the polymer formulations before processing and can influence the permeability of the coatings. This property can be used if necessary, as an additional control parameter.

### Pigments

Only rarely a pigment is added in soluble form. As a rule, pigments, such as aluminum oxide or iron oxide pigments are used in dispersed form. Titanium dioxide is used as a whitening pigment. Standard proportions for use of pigments are between 10 - 200, 20 - 200 % by weight relative to the total weight of the at least one polymer in the coating layer. Proportions up to 200 % by weight based on the total weight of the at least one polymer can be easily processed.

In a particularly advantageous embodiment, the pigment is used directly in concentrated form as an additional outer layer a so called top coat. Application takes place in the form of powder or by spraying from aqueous suspension with 5 to 35% (w/w) solid content. The necessary concentration is lower than for incorporation into the polymer layer and amounts to 0.1 to 2% by weight relative to the weight of the pharmaceutical form.

### Optional top coats and sub coats

Optionally the hard shell capsule can be additionally coated with a sub coat or a top coat or both.

A sub coat can be located between capsule and the coating layer, comprising the at least one polymer as disclosed. A sub coat has essentially no influence on the active ingredient release characteristics but may for instance improve the adhesion of the polymer coating layer. A sub coat is preferably essentially water-soluble, for instance it may consist of substances like HPMC as a film former. The average thickness of a sub coat layer is usually very thin, for example not more than 15 µm, preferably not more than 10 µm (0.1 - 1.0 mg/cm²). A sub coat or a top coat has not necessarily to be applied on the hard shell capsule in the pre-locked state.

A top coat can be located onto the coating layer, comprising the at least one polymer as disclosed. A top coat is also preferably water-soluble or essentially water-soluble. A top coat may have the function of colouring the pharmaceutical or nutraceutical form or protecting from environmental influences for instance from moisture during storage. The top coat can consist out of a binder, for instance a water-soluble polymer like a polysaccharide or HPMC, or a sugar compound like saccharose. The top coat can further contain pharmaceutically or nutraceutically acceptable excipients like pigments, plasticizers, emulsifiers or glidants in high amounts. The topcoat has essentially no influence on the release characteristics. A top coat can be applied on top of the pharmaceutical or nutraceutical dosage form comprising the polymer-coated hard shell capsule in the final-locked state as described herein. The average thickness of a top coat layer is usually very thin, for example not more than 15 µm, preferably not more than 10 µm (0.1 - 1.0 mg/cm²).

### PROCESS FOR PREPARING A COATED HARD SHELL CAPSULE

Described is a process for preparing a polymer-coated hard shell capsule, suitable as container for biologically active ingredients, wherein the hard shell capsule comprises a body and a cap, wherein in the closed state the cap overlaps the body either in a pre-locked state or in a final-locked state, wherein the hard shell capsule is provided in the pre-locked state and spray-coated with a coating solution, suspension or dispersion according to the present invention to create a coating layer which covers the outer surface of the hard shell capsule in the pre-locked state.

In a further process step the pre-locked hard shell capsule can be provided with a fill comprising at least one biologically active ingredient and is closed to the final-locked state.

In such a further process step the polymer-coated hard shell capsule in the pre-locked state can be opened, filled with a fill comprising a biologically active ingredient, and is closed in the final-locked state. This further process step is preferably performed in that the coated hard shell capsule in the pre-locked state is provided to a capsule-filling machine, which performs the opening, filling with a fill comprising at least one biologically active ingredient and closing of the polymer-coated hard shell capsule to the final-locked state.

This further process step results in a final-locked polymer-coated hard shell capsule, which is a container for at least one biologically active ingredient. The final-locked polymer-coated hard shell capsule, which as a container for at least one biologically active ingredient is a pharmaceutical or nutraceutical dosage form.

The dosage form preferably comprises a polymer-coated hard shell capsule in the final-locked state containing a fill comprising at least one biologically active ingredient, wherein the polymer-coated hard shell capsule comprises a coating layer according to the invention, where the coating layer covers the outer surface area of the capsule in the pre-locked state but not the overlapping area where the cap covers the body in the pre-locked state.

A coating suspension comprising the at least one polymer, the at least one glidant and the at least one emulsifier can contain an organic solvent, for instance acetone, iso-propanol or ethanol. The concentration of dry weight material in the organic solvent can be about from 5 to 50 % by weight of polymer. A suitable spraying concentration can be about 5 to 25 % by dry weight.

A coating suspension can be the dispersion of the at least one polymer, the at least one glidant and the at least one emulsifier in an aqueous medium, for instance water or a mixture of 80 % by weight or more of water and 20 % or less by weight of water-soluble solvents, such as acetone or isopropanol. A suitable concentration of dry weight material in the aqueous medium can be from about 5 to 50 % by weigh. A suitable spraying concentration can be about 5 to 25 % by dry weight.

The spray coating is preferably performed by spraying the coating solution or dispersion onto the pre-locked capsules in a drum coater or in a fluidized bed coating equipment.

The surface tension of the coating solution, suspension or dispersion is preferably determined according to the measurement as described in example 1.

In another preferred embodiment, the coating solution, suspension or dispersion is having a surface tension in the range of 5 to 38 mN/m, or 5 to 36 mN/m, or 5 to 33 mN/m; more preferably the coating solution, suspension or dispersion is having a surface tension in the range of 20 to 36 mN/m, or 20 to 38 mN/m, or 20 to 33 mN/m; and most preferably the coating solution, suspension or dispersion is having a surface tension in the range of 25 to 33 mN/m, or 25 to 38 mN/m, or 25 to 36mN/m; each case measured in accordance to DIN EN 14370:2004.

### Process for preparing a fill for the dosage form

Suitable processes for preparing the fill for the pharmaceutical or nutraceutical dosage form are well known to a skilled person. A suitable pprocess for preparing the fill for the pharmaceutical or nutraceutical dosage form as disclosed herein can be by forming a core comprising the biologically active ingredient in the form of pellets by direct compression, compression of dry, wet or sintered granules, by extrusion and subsequent rounding off, by wet or dry granulation, by direct pelleting or by binding powders onto active ingredient-free beads or neutral cores or active ingredient-containing particles or pellets and optionally by applying coating layers in the form of aqueous dispersions or organic solutions in spray processes or by fluidized bed spray granulation.

### CAPSULE FILLING MACHINE

The polymer-coated hard shell capsule is provided in the pre-locked state to a capsule-filling machine, which performs the steps of separating the body and the cap, filling the body with the fill and rejoining the body and the cap in the final-locked state.

The capsule filling machine used can be a capsule filling machine, preferably a fully automated capsule filling machine, that is capable to produce filled and closed capsules at a speed with an output of 1,000 or more filled and finally closed capsules per hour. Capsule filling machines, preferably fully automated capsule filling machines, are well known in the art and commercially available from several companies. A suitable capsule filling machine as used in the examples can be for instance ACG, model AFT Lab.

The capsule filling machine used can be preferably operated at a speed with an output of 1,000 or more, preferably 10,000 or more, 100,000 or more, 10,000 up to 500,000, filled and finally closed capsules per hour.

### CAPSULE FILLING MACHINE GENERAL OPERATIONS

Before the capsule filling process, the capsule filling machine is provided with a sufficient number or amount of pre-coated hard shell capsules in the pre-locked state. The capsule filling machine is also provided with enough amounts of fill to be filled in during operation.

The hard shell capsules in the pre-locked state may fall by gravity into feeding tubes or chutes. The capsules can be uniformly aligned by mechanically gauging the diameter differences between the cap and the body. The hard shell capsules are then usually fed, in proper orientation, into a two-section housing or brushing.

The diameter of the upper bushing or housing is usually larger than the diameter of the capsule body bushing; thus, the capsule cap can be retained within an upper bushing while the body is pulled into a lower bushing by vacuum. Once the capsule is opened/ the body and the cap are separated, the upper and lower housing or bushing are separated to position the capsule body for filling.

The open capsule body is then filled with the fill. Various types of filling mechanisms can be applied, with respect to the different fillings such as granules, powders, pellets or mini-tablets. Capsule filling machines in general employ a variety of mechanisms to handle the various dosage ingredients as well as various numbers of filling stations. The dosing systems are usually based on volumetric or amounts of fills governed by the capsule size and capacity of the capsule body. The empty capsule manufacturers usually provide reference tables that indicate the volume capacity of their capsule body and the maximum fill weight for different capsule sizes based on the density of the fill material. After the filling, the body and the cap are rejoined by the machine in the final-locked state or position.

### USE / METHOD OF USE / METHOD STEPS

The process for preparing a polymer-coated hard shell capsule suitable as described herein can be understood as a method of use of a hard shell capsule comprising a body and a cap, wherein in the closed state the cap overlaps the body either in a pre-locked state or in a final-locked state, for preparing a polymer-coated hard shell capsule, suitable as container for pharmaceutical or nutraceutical biologically active ingredients, comprising the steps of
a) providing the hard shell capsule is provided in the pre-locked state and
b) spray-coating with a coating solution, suspension or dispersion comprising a polymer or a mixture of polymers to create a coating layer which covers the outer surface of the hard shell capsule in the pre-locked state.

The spray-coating can be preferably applied by using a drum coater equipment or a fluidized bed coating equipment. A suitable product temperature during the spray-coating process can be in the range from about 15 to 40, preferably from about 20 to 35 °C. A suitable spray rate can be in the range from about 0.3 to 17.0, preferably 0.5 to 14 [g/min/kg]. After spray-coating a drying step is included.

The polymer-coated hard shell capsule in the pre-locked state can be opened in a step c), filled with a fill comprising a pharmaceutical or a nutraceutical biologically active ingredient in a step d), and is then closed in a step e) to the final-locked state.

Steps c) to e) can be performed manually or preferably supported by a suitable equipment, for instance a capsule-filling machine. Preferably, the coated hard shell capsule in the pre-locked state is provided to a capsule-filling machine, which performs the opening step c), the filling with a fill comprising a pharmaceutical or a nutraceutical biologically active ingredient in step d) and the closing of the capsule to the final-locked state in step e).

The selection of the processes in all their generic or specific features and embodiments as disclosed herein can be combined without restriction with any other generic or specific selections of materials or numerical features and embodiments as disclosed herein, such as polymers, capsule materials, capsule sizes, coating thicknesses, biologically active ingredients and any other embodiments as disclosed.

### PHARMACEUTICAL OR NUTRACEUTICAL DOSAGE FORM

Disclosed is a pharmaceutical or nutraceutical dosage form comprising a polymer-coated hard shell capsule in the final-locked state containing a fill comprising a pharmaceutical or nutraceutical biologically active ingredient, wherein the polymer-coated hard shell capsule comprises a coating layer comprising a polymer or a mixture of polymers, where the coating layer covers the outer surface area of the capsule in the pre-locked state. Since the outer surface area of the capsule in the pre-locked state is larger than outer surface area of the capsule in the final-locked state a part of the polymer coating layer is hidden or enclosed between the body and the cap of the hard shell capsule, which provides an efficient sealing.

### ITEMS

In particular, the present invention refers to:
1. Process for preparing a polymer-coated hard shell capsule, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and coated, preferably spray-coated, with a coating solution, suspension or dispersion comprising or consisting of
   a) at least one polymer, preferably at least one (meth)acrylate copolymer, anionic cellulose, ethyl cellulose or starch comprising at least 35 % by weight amylose;
   b) at least one glidant;
   c) at least one emulsifier;
   d) optionally at least one plasticizer;
   e) optionally at least one biologically active ingredient, preferably at least one pharmaceutical, nutraceutical; or at least one cosmetic ingredient; and
   f) optionally at least one additive, different from a) to e);
   to obtain a coated, preferably only at the outer surface, hard shell capsule in the pre-locked state, wherein the coating solution, suspension or dispersion has a surface tension of at most 38 mN/m, preferably at most 36 mN/m, more preferably at most 33 mN/m.
2. Process according to item 1, wherein the base material of the body and the cap is selected from hydroxypropyl methyl cellulose, starch, gelatin, pullulan and a copolymer of a C1- to C4-alkylester of (meth)acrylic acid and (meth)acrylic acid, preferably is hydroxypropyl methyl cellulose.
3. Process according to item 1 or 2, wherein the at least one polymer is selected from at least one (meth)acrylate copolymer; preferably having a glass transition temperatures *T_{gm}* of 125 °C or less.
4. Process according to item 1 or 2, wherein the at least one polymer is
   i) a Core-Shell polymer, which is a copolymer obtained by a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid; or
   ii) an anionic polymer obtained by polymerizing 25 to 95 % by weight C1- to C12-alkyl esters of acrylic acid or of methacrylic acid and 75 to 5% by weight (meth)acrylate monomers with an anionic group; or
   iii) a cationic (meth)acrylate copolymer obtained by polymerizing C1- to C4-alkyl esters of acrylic or of methacrylic acid and an alkyl ester of acrylic or of methacrylic acid with a tertiary or a quaternary ammonium group in the alkyl group; or
   iv) a (meth)acrylate copolymer obtained by polymerizing methacrylic acid and ethyl acrylate, methacrylic acid and methyl methacrylate, ethyl acrylate and methyl methacrylate or methacrylic acid, methyl acrylate and methyl methacrylate; or
   v) a (meth)acrylate copolymer obtained by polymerizing 40 to 60 % by weight of methacrylic acid and 60 to 40 % by weight of ethyl acrylate; or
   vi) a (meth)acrylate copolymer obtained by polymerizing 60 to 80 % of ethyl acrylate and 40 to 20 % by weight of methyl methacrylate; or
   vii) a (meth)acrylate copolymer obtained by polymerizing 5 to 15 % by weight methacrylic acid, 60 to 70 % by weight of methyl acrylate and 20 to 30 % by weight methyl methacrylate; or mixtures thereof.
5. Process according to item 1 or 2, wherein the at least one polymer is a mixture of
   i) a (meth)acrylate copolymer obtained by copolymerizing 40 to 60 % by weight of methacrylic acid and 60 to 40 % by weight of ethyl acrylate and a (meth)acrylate copolymer obtained by polymerizing 60 to 80 % of ethyl acrylate and 40 to 20 % by weight of methyl methacrylate at a ratio from 10:1 to 1:10 by weight; or
   ii) a (meth)acrylate copolymer obtained by copolymerizing 5 to 15 % by weight methacrylic acid, 60 to 70 % by weight of methyl acrylate and 20 to 30 % by weight methyl methacrylate and a (meth)acrylate copolymer obtained by copolymerizing 40 to 60 % by weight of methacrylic acid and 60 to 40 % by weight of ethyl acrylate at a ratio from 1:1 to 5:1 by weight.
6. Process according to item 1 or 2, wherein the at least one polymer is selected from at least one anionic cellulose, ethyl cellulose or starch comprising at least 35 % by weight amylose.
7. Process according to item 1 or 2, wherein the at least one polymer is selected from starch, alginates or salts of alginates, sodium alginate, pectin, shellac, zein, carboxymethyl-zein, modified starch, marine sponge collagen, chitosan, gellan gum, ethyl cellulose and pectin, modified starch and alginate and/or pectin, shellac and alginate and/or pectin, shellac and inulin, whey protein and gums, zein, sodium alginate and chitosan or mixtures thereof.
8. Process according to any of the preceding items, wherein the at least one glidant
   i) is present in an amount of 3 to 75 % by weight, based on the total weight of the at least one polymer a) and/or
   ii) is selected from silica, ground silica, fumed silica, kaolin calcium silicate, magnesium silicate, colloidal silicone dioxide, talc, stearate salts, sodium stearyl fumarate, starch, stearic acid or mixtures thereof, preferably talc, magnesium stearate, colloidal silicon dioxide and glycerol monostearate or mixtures thereof, more preferred glycerol monostearate and talc.
9. Process according to any of the preceding items, wherein the at least one emulsifier
   i) is present in an amount of 1.5 to 40 % by weight, based on the total weight of the at least one polymer a) and/or
   ii) is a non-ionic emulsifier, preferably a non-ionic emulsifier having an HLB > 10.
10. Process according to any of the preceding items, wherein the at least one plasticizer
   i) is present in an amount of 2 to 40 % by weight, based on the total weight of the at least one polymer a) and/or
   ii) is selected from alkyl citrates, alkyl phthalates, alkyl sebacates, polyethylene glycols, polypropylene glycols or mixtures thereof, preferably diethyl sebacate, triethyl citrate (TEC), acetyl triethyl citrate (ATEC), diethyl sebacate, dibutyl sebacate (DBS), polyethylene glycols or mixtures thereof.
11. Process according to any of the preceding items, wherein up to 400 % by weight, preferably up to 200 % by weight, more preferably up to 100 % by weight or up to 50 % by weight, or up to 30 % by weight, or up to 15 % by weight, or up to 5 % by weight or up to 3 % by weight, or up to 1 % by weight, on the total weight of the at least one polymer a), of at least one additive are comprised; preferably selected from antioxidants, brighteners, flavouring agents, flow aids, fragrances, penetration-promoting agents, pigments, polymers different from a), pore-forming agents or stabilizers, or combinations thereof.
12. Process according to any of the preceding items, wherein the body and the cap are comprising encircling notches or dimples in the area where the cap overlaps the body, that allow the capsule to be closed by a snap-into-place mechanism either in the pre-locked state or in the final-locked state.
13. Process according to any of the preceding items, wherein the body comprises a tapered rim.
14. Process according to any of the preceding items, wherein the coating layer is applied in an amount of about 0.7 to 20 mg/cm², preferably 2 to 10, 4 to 8, 1.0 to 8, 1.5 to 5.5, or 1.5 to 4 mg/cm².
15. Process according to any of the preceding items, wherein the polymer-coated hard shell capsule in the pre-locked state is opened, filled with a fill comprising a pharmaceutical or a nutraceutical biologically active ingredient, and is closed to the final-locked state.
16. Process according to any of the preceding items, wherein the polymer-coated hard shell capsule in the pre-locked state is provided to a capsule-filling machine, which performs opening, filling with a fill comprising a pharmaceutical or a nutraceutical biologically active ingredient and closing to the final-locked state.
17. Polymer-coated hard shell capsule, obtained from a process according to any of items 1 to 16.
18. Use of the polymer-coated hard shell capsule according to item 17 for immediate, delayed or sustained release, preferably delayed release, more preferably for immediate, delayed or sustained release for intestine delivery.

### EXAMPLES

### Preparation of compositions

### EXAMPLE 1: MEASUREMENT OF COATING SUSPENSION SURFACE TENSION

In example 1 the surface tension is measured for several coating suspensions. It is indicated in the respective table (tables 1 to 3), if the coating suspension is according to the invention with "invent" or if the coating suspension is not according to the invention with "comp."

### Principle of the test method (according to DIN EN 14370:2004)

The free surface enthalpy per unit of surface area is referred to as surface tension. It is given in mN/m. The methods are based on the measurement of the maximum force, which is necessary to exert vertically on a plate, in contact with the surface of the liquid, in order to separate it from the surface. The method is applicable to aqueous solutions of most substances regardless of their degree of purity.

The plate is suspended vertically from a metal pin and a wire mounting bracket to establish the connection to the force measuring system. The measurement vessel holding the test solution is a temperature-controlled glass vessel. It is designed so that, during the measurement, the temperature of the test solution and the gas phase above its surface remains constant and that the sample cannot evaporate.

### Apparatus:

Tensiometer: Kruess Processor Tensiometer K12 (Kruess)

### Parameters:

Temperature: 30 °C
Heating time: 15 min

**Table 1: Surface Tension Results (1 of 3)**

| **Surface Tension Measurements** | | | | | | | | | | | |
|---|---|---|---|---|---|---|---|---|---|---|---|
| Formulation No. | | 1 | 2 | 3 | 4 | 5 | 6 | 7 | 8 | 9 | 10 |
| EUDRAGIT^{®} L 30 D-55 | g | 0.00 | 90.00 | 90.00 | - | - | - | - | 90.00 | 80.00 | 80.00 |
| EUDRAGIT^{®} NM 30 D | g | 10.00 | 10.00 | 10.00 | - | - | - | - | 10.00 | | |
| EUDRAGIT^{®} FS 30 D | g | - | - | - | - | - | - | - | - | 20.00 | 20.00 |
| METHOCEL^{™} VLV Premium | g | - | - | - | 90.00 | 90.00 | 90.00 | 90.00 | - | - | - |
| Triethyl citrate | g | 6.00 | 6.00 | 6.00 | - | 5.29 | 5.29 | 5.29 | 6.00 | 6.00 | 6.00 |
| Glycerol monostearate (40-55%) | g | - | 3.00 | 1.50 | - | 2.65 | 2.65 | 2.65 | 3.00 | 3.00 | 3.00 |
| Polysorbate 80 | g | - | 1.20 | 0.60 | - | 1.06 | 1.06 | 1.06 | 3.00 | 3.00 | 1.20 |
| Water | g | 254.00 | 291.80 | 272.90 | 810.00 | 891.00 | 1221.00 | 1881.01 | 308.0 | 308.0 | 251.00 |
| Total | g | 360.00 | 402.00 | 381.00 | 900.00 | 990.00 | 1320.00 | 1980.01 | 420.00 | 420.00 | 402.00 |
| Solid content | % | 10.00 | 10.00 | 10.00 | 10.00 | 10.00 | 7.50 | 5.00 | 10.00 | 10.00 | 10.00 |
| Emulsifier Concentration [w/w] | % | 0.00 | 0.30 | 0.16 | 0.00 | 0.11 | 0.08 | 0.05 | 0.71 | 0.71 | 0.30 |
| Surface Tension | mN/m | 41.3 | 32.6 | 31.8 | 44.4 | 33.0 | 33.0 | 32.6 | 31.5 | 31.0 | 30.2 |
| Status | - | comp. | invent | invent | comp. | invent | invent | invent | invent | invent | invent |

**Table 2 Surface Tension Results (2 of 3)**

| **Surface Tension Measurements** | | | | | | | | | | |
|---|---|---|---|---|---|---|---|---|---|---|
| Formulation No. | | 11 | 12 | 13 | 14 | 15 | 16 | 17 | 18 | 19 |
| EUDRAGIT^{®} L 30 D-55 | g | 0.00 | 90.00 | 90.00 | 50.00 | 50.00 | - | - | - | - |
| EUDRAGIT^{®} NM 30 D | g | 10.00 | 10.00 | 10.00 | - | - | - | - | - | - |
| EUDRAGIT^{®} FS 30 D | g | - | - | - | 50.00 | 50.00 | - | - | - | - |
| METHOCEL^{™} VLV Premium | g | - | - | - | - | - | 90.00 | 90.00 | 90.00 | 90.00 |
| Triethyl citrate | g | 6.00 | 6.00 | 6.00 | 6.00 | 6.00 | 10.59 | 5.29 | 9.79 | 9.79 |
| Glycerol monostearate (40-55%) | g | - | 3.00 | 1.50 | 3.00 | 3.00 | 5.29 | 2.65 | 2.65 | 2.65 |
| Polysorbate 80 | g | - | 1.20 | 0.60 | 3.00 | 1.20 | 2.12 | 4.56 | 1.06 | 4.56 |
| Water | g | 74.00 | 90.80 | 82.40 | 308.0 | 90.80 | 972.01 | 931.50 | 931.50 | 972.00 |
| Total | g | 180.00 | 201.00 | 192.41 | 420.00 | 201.00 | 1080.01 | 1035.00 | 1035.00 | 1080.00 |
| Solid content | % | 20.00 | 20.00 | 20.00 | 10.00 | 20.00 | 10.00 | 10.00 | 10.00 | 10.00 |
| Emulsifier Concentration [w/w] | % | 0.00 | 0.60 | 0.31 | 0.71 | 0.60 | 0.20 | 0.54 | 0.10 | 0.51 |
| Surface Tension | mN/m | 39.4 | 33.5 | 31.6 | 34.2 | 29.8 | 30.4 | 32.7 | 31.2 | 32.0 |
| Status | - | comp. | invent | invent | invent | invent | invent | invent | invent | invent |

**Table 3: Surface Tension Results (3 of 3)**

| **Surface Tension Measurements** | | | | | | | |
|---|---|---|---|---|---|---|---|
| Formulation No. | | 20 | 21 | 22 | 23 | 24 | 25 |
| EUDRAGIT^{®} L 30 D-55 | g | 00.00 | - | - | 50.00 | - | - |
| EUDRAGIT^{®} NM 30 D | g | - | 100.00 | - | - | 500.00 | - |
| EUDRAGIT^{®} FS 30 D | g | - | - | 100.00 | - | - | 50.00 |
| Water | g | - | - | - | 100.00 | 100.00 | 100.00 |
| Total | g | 100.00 | 100.00 | 100.00 | 150.00 | 150.00 | 150.00 |
| Solid content | % | 30.00 | 30.00 | 30.00 | 10.00 | 10.00 | 10.0 |
| Emulsifier Concentration [w/w] | % | 0.00 | 0.00 | 0.00 | 0.00 | 0.00 | 0.00 |
| Surface Tension | mN/m | 39.0 | 40.0 | 46.4 | 40.9 | 39.9 | 45.8 |
| Status | - | comp. | comp. | comp. | comp. | comp. | comp. |

### EXAMPLE 2: (COMPARATIVE) ENTERIC COATING OF PRE-LOCKED CAPSULES IN DRUM COATER

The functional and top coat formulations are calculated considering a surface area in pre-locked state of 594.5 mm² and a batch size of 400,000 capsules (K-caps Size 0).

### Functional Coating:

EUDRAGIT^{®} L 30 D-55 was provided as 30 % by weight aqueous polymer dispersion and diluted with the calculate water amount. The dispersion was gently stirred while Triethyl citrate was added. After 10 minutes the EUDRAGIT^{®} NM 30D was added slowly under continuous stirring. The final coating suspension was sieved throughout a 400µm sieve and stirred during the coating process. The capsules were coated in the pre-locked state utilizing a drum coater.

**Table 4: Functional Coating - Formulation 1 of Example 1**

| **Material** | **Composition** | **Solid Composition Percentage** |
|---|---|---|
| EUDRAGIT^{®} L 30 D-55 | 1.8 mg/cm² | 75.00 % |
| EUDRAGIT^{®} NM 30 D | 0.2 mg/cm² | 8.33 % |
| Triethyl citrate | 20 % on ds* | 16.67 % |
| Demineralized Water | On demand | n/a |
| | | |
| Solid content | 10% w/w | |
| Total solid weight gain | 2.4 mg/cm² | |

| | | |
|---|---|---|
| *Quantity based on dry polymer substance [%] | | |

### Top Coating

METHOCEL^{™} was thoroughly dispersed in the water while gently stirring to prevent lumping. The spraying suspension was gently stirred during the coating process.

**Table 5: Top Coating - Formulation 4 of Example 1**

| **Material** | **Composition** | **Solid Composition Percentage** |
|---|---|---|
| METHOCEL^{™} E3 | 0.5 mg/cm² | 100.00 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules are coated in a fully perforated side-vended pan coating system Bohle BFC 400. The relevant process parameters are listed in Table 6.

**Table 6: Process Parameter**

| **Parameter** | **Value** | **Value** |
|---|---|---|
| | Functional Coating | Top Coating |
| Machine | Bohle BFC 400 | |
| Batch size [kg] | 43.6 | |
| Nozzle bore [mm] | 0.8 | |
| Number of Nozzles | 4 | |
| Spray Gun to Product Bed Distances [cm] | 29 | |
| Spray rate [g/min] | 120 | 120 |
| Exhaust air temperature [°C] | 28 - 29 | 28 |
| Inlet air temperature [°C] | 31 - 33 | 30 |
| Inlet air volume [m³/h] | 3000 | 3000 |
| Atomizing pressure [bar] | 0.8 - 1.1 | 1.1 |
| Flat pattern pressure [bar] | 0.8 - 1.1 | 1.1 |
| Pan speed [rpm] | 7 | 7 |
| Product temperature [°C] | 28-29 | 28 |
| LOD [%] | 3.82 - 5.01 | 3.76 - 4.28 |
| Process time [min] | 465 | 187 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Results:

At the end of the functional coating 22.7 % and at the end of the top coating process 17.3 - 26.3% (n=5) of the batch showed bridging.

### EXAMPLE 3: (COMPARATIVE) ENTERIC COATING OF PRE-LOCKED CAPSULES IN DRUM COATER

The functional and top coat formulations are calculated considering a surface area in pre-locked state of 594.5 mm² and a batch size of 200,000 capsules (K-caps Size 0).

### Functional Coating:

EUDRAGIT^{®} L 30 D-55 was provided as 30 % by weight aqueous polymer dispersion and diluted with the calculate water amount. The dispersion was gently stirred while Triethyl citrate was added. After 10 minutes the EUDRAGIT^{®} NM 30D was added slowly under continuous stirring. The final coating suspension was sieved throughout a 400µm sieve and stirred during the coating process. The capsules were coated in the pre-locked state utilizing a drum coater.

**Table 7: Functional Coating - Formulation 1 of Example 1**

| **Material** | **Composition** | **Solid Composition Percentage** |
|---|---|---|
| EUDRAGIT^{®} L 30 D-55 | 1.8 mg/cm² | 75.00 % |
| EUDRAGIT^{®} NM 30 D | 0.2 mg/cm² | 8.33 % |
| Triethyl citrate | 20 % on ds* | 16.67 % |
| Demineralized Water | On demand | n/a |
| | | |
| Solid content | 10% w/w | |
| Total solid weight gain | 2.4 mg/cm² | |

| | | |
|---|---|---|
| *Quantity based on dry polymer substance [%] | | |

### Top Coating

METHOCEL^{™} E3, products are carbohydrate polymers which dissolve in cold water by swelling and subsequent hydration. METHOCEL^{™} E3 is thoroughly dispersed in the water while gently stirring to prevent lumping. The spraying suspension was gently stirred during the coating process.

**Table 8: Top Coating - Formulation 4 of Example 1**

| **Material** | **Composition** | **Solid Composition Percentage** |
|---|---|---|
| METHOCEL^{™} E3 | 0.5 mg/cm² | 100.00 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules are coated in a fully perforated side-vended pan coating system Bohle BFC 200. The relevant process parameters are listed in Table 9.

**Table 9: Process Parameter**

| **Parameter** | **Value** | **Value** |
|---|---|---|
| | Functional Coating | Top Coating |
| Machine | Bohle BFC 200 | |
| Batch size [kg] | 19.2 | |
| Nozzle bore [mm] | 0.8 | |
| Number of Nozzles | 2 | |
| Spray Gun to Product Bed Distances [cm] | 15 | |
| Spray rate [g/min] | 60-78 | 60 |
| Exhaust air temperature [°C] | 30 | 30 |
| Inlet air temperature [°C] | 33 - 38 | 35 |
| Inlet air volume [m³/h] | 1500 | 1500 |
| Atomizing pressure [bar] | 1.0 | 1.1 |
| Flat pattern pressure [bar] | 1.2 | 1.1 |
| Pan speed [rpm] | 7-10 | 7 |
| Product temperature [°C] | 28-30 | 29 |
| LOD [%] | 2.38 - 3.46 | 2.57 - 2.92 |
| Process time [min] | 335 | 180 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Results:

After application of the functional and top coating process above 50% (n=5) of the batch showed bridging.

### Example 4: (Comparative) Enteric coating of pre-locked capsules in drum coater

The functional coat formulations are calculated considering a surface area in pre-locked state of 594.5 mm² and a batch size of 30,000 capsules (K-caps Size 0).

### Functional Coating

EUDRAGIT^{®} L 30 D-55 was provided as 30 % by weight aqueous polymer dispersion and diluted with the calculate water amount. The dispersion was gently stirred while Triethyl citrate was added. After 10 minutes the EUDRAGIT^{®} NM 30D was added slowly under continuous stirring. The final coating suspension was sieved throughout a 400µm sieve and stirred during the coating process. The capsules were coated in the pre-locked state utilizing a drum coater.

**Table 10: Functional Coating - Formulation 1 of Example 1**

| **Material** | **Composition** | **Solid Composition Percentage** |
|---|---|---|
| EUDRAGIT^{®} L 30 D-55 | 1.8 mg/cm² | 75.0 % |
| EUDRAGIT^{®} NM 30D | 0.2 mg/cm² | 8.3% |
| Triethyl citrate | 20.0 % on ds* | 16.7 % |
| Demineralized Water | On demand | n/a |
| | | |
| Solid content | 7.5% w/w | |
| Total solid weight gain | 2.4 mg/cm² | |
| Total applied polymer | 2.0 mg/cm² | |

| | | |
|---|---|---|
| *Quantity based on dry polymer substance [%] | | |

### Capsule Coating Process

The capsules are coated in a fully perforated side-vended pan coating system Glatt GMPC 2. The relevant process parameters are listed in Table 11.

**Table 11: Process Parameter**

| **Parameter** | **Value** |
|---|---|
| Machine | Glatt GMPC 2 |
| Batch size [g] | 3,288 |
| Nozzle bore [mm] | 1.0 |
| Internal tube diameter [mm] | 3.0 |
| Peristaltic pump | Watson Marlow |
| Atomizing pressure [bar] | 1.2 |
| Flat pattern pressure [bar] | 1.9 |
| Pan speed [rpm] | 7 |
| Inlet air volume [m³/h] | 500 |
| Inlet air temperature [°C] | 45 - 50 |
| Inlet air humidity [g/m³] | 3.4 |
| Exhaust air temperature [°C] | 34.8 - 37.5 |
| Product temperature [°C] | 29.5 - 30.8 |
| Spray rate [g/min/kg] | 6.2 - 10.0 |
| Process time [min] | 217 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Result:

Result of this example 15/100 need high forces to sperate capsule cap and body.

### Example 5: (Inventive) Enteric coating of pre-locked capsules in drum coater

The functional and top coat formulations are calculated considering a surface area in pre-locked state of 594.5 mm² and a batch size of 40,000 capsules (K-caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 12: Functional Coating - Formulation 2 of Example 1**

| **Material** | **Composition** | **Solid Composition Percentage** |
|---|---|---|
| EUDRAGIT^{®} L 30 D-55 | 1.8 mg/cm² | 67.2 % |
| EUDRAGIT^{®} NM 30 D | 0.2 mg/cm² | 7.5% |
| Triethyl citrate | 20.0 % on ds* | 14.9 % |
| Glycerol monostearate | 10.0 % on ds* | 7.5 % |
| Polysorbate 80 | 4.0 % on ds* | 3.0 % |
| Demineralized Water | On demand | n/a |
| | | |
| Solid content | 10.0% w/w | |
| Total solid weight gain | 2.7 mg/cm² | |
| Total applied polymer | 2.0 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 13: Top Coating - Formulation 5 of Example 1**

| **Material** | **Composition** | **Solid Composition Percentage** |
|---|---|---|
| METHOCEL^{™} VLV | 0.5 mg/cm² | 90.9 % |
| Glycerol monostearate | 5.9 % on ds* | 5.4 % |
| Triethyl citrate | 2.9 % on ds* | 2.6 % |
| Polysorbat 80 | 1.2 % on ds* | 1.1 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules are coated in a fully perforated side-vended pan coating system Bohle BFC 40. The relevant process parameters are listed in Table .

The equipment parameters were kept equal for functional and top coating.

**Table 14: Process Parameter**

| **Parameter** | **Value** |
|---|---|
| Machine | Bohle BFC 40 |
| Batch size [g] | 4360 |
| Nozzle bore [mm] | 1.0 |
| Atomizing pressure [bar] | 1.0 |
| Flat pattern pressure [bar] | 1.2 |
| Pan speed [rpm] | 8 |
| Inlet air volume [m³/h] | 500 |
| Inlet air temperature [°C] | 38 - 43 |
| Exhaust air temperature [°C] | 30 |
| Product temperature [°C] | 29 - 30 |
| Spray rate [g/min/kg] | 5.7 - 8.0 |
| Process time [min] | 200 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Results:

Result of this example 2/100 need high forces to sperate capsule cap and body.

### Disintegration Test (according to European Pharmacopeia 2.9.1 Test B modified method based on gastro-resistant capsules) - Capsule unfilled

| | |
|---|---|
| Method: | 2h 0.1N HCl followed by full change Buffer system pH 6.8. |
| Apparatus: | PTZ Auto 4 EZ Pharma Test |
| Detection method: | Visually and electrical Impedance |
| Temperature: | 37.0°C |
| Media I: | 700 ml 0.1 N HCL according to European Pharmacopeia |
| Media II: | 700mL pH to 6.8 Phosphate buffer according to European Pharmacopeia |
| Samples: | n=6 |

**Table 15: Disintegration Results**

| Media | Time [min] | Sub Sample 1 n=3 | Sub Sample 2 n=3 |
|---|---|---|---|
| 0.1N HCl | Start | intact | intact |
| 0.1N HCl | 60 minutes | intact | intact |
| 0.1N HCl | 120 minutes | intact | intact |
| pH 6.8 | 5 minutes | release | release |

### Dissolution Test (according to European Pharmacopeia (2.9.3) apparatus II)

Capsule manually filled. The polymer coated pre-locked capsules were manually filled with 500mg Caffeine/Lactose Mixture 4:6, closed to the final-locked state and tested in a dissolution test.

| | |
|---|---|
| Method: | |
| Apparatus: | ERWEKA DT 700 Paddle Apparatus (USP II) |
| Detection method: | Online UV |
| Temperature: | 37.5°C |
| Media I: | 700 ml 0.1 N HCL adjusted to pH 1.2 (by using 2N NaOH and 2N HCl) |
| Media II: | After 2hours in media I 214 ml 0.2 N Na₃PO₄ solution added to increase pH to 6.8 (fine adjustment of pH by using 2N NaOH and 2N HCl) |
| Paddle speed: | 75rpm |

**Table 16: Dissolution Results**

| Media | Time [min] | Sample 1 [% released] | Sample 2 [% released] | Sample 3 [% released] | Sample 4 [% released] | Sample 5 [% released] | Sample 6 [% released] |
|---|---|---|---|---|---|---|---|
| 0.1N HCL | 0 | 0.04 | -0.01 | 0.00 | 0.00 | 0.00 | 0.01 |
| 0.1N HCL | 30 | 0.02 | 0.01 | 0.00 | 0.01 | 0.01 | -0.01 |
| 0.1N HCL | 60 | 0.03 | -0.01 | 0.00 | 0.01 | 0.03 | 0.01 |
| 0.1N HCL | 90 | 0.03 | 0.04 | 0.00 | 0.01 | 0.01 | 0.01 |
| 0.1N HCL | 120 | 0.04 | 0.03 | 0.01 | 0.02 | 0.04 | -0.01 |
| pH6.8 | 125 | 0.24 | 0.21 | 0.32 | 0.24 | 0.33 | 0.18 |
| pH6.8 | 130 | 2.90 | 2.24 | 0.89 | 0.92 | 6.27 | 0.63 |
| pH6.8 | 135 | 87.52 | 25.80 | 62.71 | 63.03 | 60.62 | 94.80 |
| pH6.8 | 140 | 96.36 | 92.35 | 86.98 | 94.08 | 88.04 | 95.53 |
| pH6.8 | 145 | 96.75 | 96.27 | 96.20 | 96.76 | 93.99 | 95.65 |
| pH6.8 | 150 | 96.49 | 95.97 | 96.86 | 96.72 | 95.26 | 95.60 |
| pH6.8 | 165 | 96.76 | 96.41 | 96.69 | 96.92 | 95.62 | 95.73 |
| pH6.8 | 180 | 96.61 | 96.18 | 96.73 | 97.12 | 95.59 | 95.72 |

### Example 6: (Inventive) Enteric coating of pre-locked capsules in drum coater

The functional and top coat formulations are calculated considering a surface area in pre-locked state of 545.8 mm² and a batch size of 30,000 capsules (V-caps plus Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process.

**Table 17: Functional Coating - Formulation 3 of Example 1**

| **Material** | **Composition** | **Solid Composition Percentage** |
|---|---|---|
| EUDRAGIT^{®} L 30 D-55 | 1.8 mg/cm² | 70.9 % |
| EUDRAGIT^{®} NM 30D | 0.2 mg/cm² | 7.9% |
| Triethyl citrate | 20.0 % on ds* | 15.8 % |
| Glycerol monostearate | 5.0 % on ds* | 3.9 % |
| Polysorbate 80 | 2.0 % on ds* | 1.6 % |
| Demineralized Water | On demand | n/a |
| | | |
| Solid content | 7.5% w/w | |
| Total solid weight gain | 2.5 mg/cm² | |
| Total applied polymer | 2.0 mg/cm² | |

| | | |
|---|---|---|
| *Quantity based on dry polymer substance [%] | | |

### Capsule Coating Process

The capsules are coated in a fully perforated side-vended pan coating system Bohle BFC 40. The relevant process parameters are listed in Table .

**Table 18: Process Parameter**

| **Parameter** | **Value** |
|---|---|
| Machine | Glatt GMPC 2 |
| Batch size [g] | 3288 |
| Nozzle bore [mm] | 1.0 |
| Internal tube diameter [mm] | 3.0 |
| Peristaltic pump | Watson Marlow |
| Atomizing pressure [bar] | 1.2 |
| Flat pattern pressure [bar] | 1.9 |
| Pan speed [rpm] | 7 |
| Inlet air volume [m³/h] | 500 |
| Inlet air temperature [°C] | 54 |
| Inlet air humidity [g/m³] | 3.4-3.6 |
| Exhaust air temperature [°C] | 40.4-41.2 |
| Exhaust air humidity [g/m³] | 6.9-7.1 |
| Product temperature [°C] | 29.8-30.1 |
| Spray rate [g/min/kg] | 7.4-8.8 |
| Process time [min] | 214 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Result:

Result of this example 2/100 need high forces to sperate capsule cap and body.

### Example 7: (inventive) Enteric coating of pre-locked capsules in drum coater

The functional and top coat formulations are calculated considering a surface area in pre-locked state of 594.5 mm² and a batch size of 30,000 capsules (K-caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process.

**Table 19: Functional Coating - Formulation 3 of Example 1**

| **Material** | **Composition** | **Solid Composition Percentage** |
|---|---|---|
| EUDRAGIT^{®} L 30 D-55 | 1.8 mg/cm² | 70.9 % |
| EUDRAGIT^{®} NM 30D | 0.2 mg/cm² | 7.9% |
| Triethyl citrate | 20.0 % on ds* | 15.8 % |
| Glycerol monostearate | 5.0 % on ds* | 3.9 % |
| Polysorbate 80 | 2.0 % on ds* | 1.6 % |
| Demineralized Water | On demand | n/a |
| | | |
| Solid content | 7.5% w/w | |
| Total solid weight gain | 2.5 mg/cm² | |
| Total applied polymer | 2.0 mg/cm² | |

| | | |
|---|---|---|
| *Quantity based on dry polymer substance [%] | | |

### Capsule Coating Process

The capsules are coated in a fully perforated side-vended pan coating system Glatt GMPC 2. The relevant process parameters are listed in Table .

**Table 20: Process Parameter**

| **Parameter** | **Value** |
|---|---|
| Machine | Glatt GMPC 2 |
| Batch size [g] | 3,288 |
| Nozzle bore [mm] | 1.0 |
| Internal tube diameter [mm] | 3.0 |
| Peristaltic pump | Watson Marlow |
| Atomizing pressure [bar] | 1.2 |
| Flat pattern pressure [bar] | 1.9 |
| Pan speed [rpm] | 7 |
| Inlet air volume [m³/h] | 500 |
| Inlet air temperature [°C] | 50 - 53.4 |
| Inlet air humidity [g/m³] | 5.9 - 6.6 |
| Exhaust air temperature [°C] | 39.4 - 40.9 |
| Exhaust air humidity [g/m³] | 9.2-10.2 |
| Product temperature [°C] | 30.5 - 32.0 |
| Spray rate [g/min/kg] | 6.3 - 7.1 |
| Process time [min] | 274 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Result:

Result of this example 1/100 need high forces to sperate capsule cap and body.

### Example 8: (Inventive) Enteric coating of pre-locked capsules in drum coater

The functional and top coat formulations are calculated considering a surface area in pre-locked state of 545.8 mm² and a batch size of 200,000 capsules (Vcaps plus Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 21: Functional Coating - Formulation 2 of Example 1**

| **Material** | **Composition** | **Solid Composition Percentage** |
|---|---|---|
| EUDRAGIT^{®} L 30 D-55 | 3.15 mg/cm² | 67.2 % |
| EUDRAGIT^{®} NM 30 D | 0.35 mg/cm² | 7.5 % |
| Triethyl citrate | 20.0 % on ds* | 14.9 % |
| Glycerol monostearate | 10.0 % on ds* | 3.0 % |
| Polysorbate 80 | 4.0 % on ds* | 7.5 % |
| Demineralized Water | On demand | n/a |
| | | |
| Solid content | 10.0% w/w | |
| Total solid weight gain | 4.7 mg/cm² | |
| Total applied polymer | 3.5 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 22: Top Coating - Formulation 5 of Example 1**

| **Material** | **Composition** | **Solid Composition Percentage** |
|---|---|---|
| METHOCEL^{™} VLV | 0.45 mg/cm² | 90.9 % |
| Glycerol monostearate | 5.9 % on ds* | 5.4 % |
| Triethyl citrate | 2.9 % on ds* | 2.6 % |
| Polysorbat 80 | 1.2 % on ds* | 1.1 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules are coated in a fully perforated side-vended pan coating system Bohle BFC 200. The relevant process parameters are listed in Table .

The equipment parameters were kept equal for functional and top coating.

**Table 23: Process Parameter**

| **Parameter** | **Value** | **Value** |
|---|---|---|
| | Functional Coating | Top Coating |
| Machine | Bohle BFC 200 | |
| Batch size [kg] | 19 | |
| Nozzle bore [mm] | 1.2 | |
| Number of Nozzles | 3 | |
| Spray Gun to Product Bed Distances [cm] | 20 | |
| Spray rate [g/min] | 70-80 | 70-75 |
| Exhaust air temperature [°C] | 30 | 30 |
| Inlet air temperature [°C] | 37 - 42 | 37-41 |
| Inlet air volume [m³/h] | 1500 | 1500 |
| Atomizing pressure [bar] | 1.0 | 1.0 |
| Flat pattern pressure [bar] | 1.2 | 1.2 |
| Pan speed [rpm] | 9 | 9 |
| Product temperature [°C] | 29 - 31 | 30-31 |
| LOD [%] | 3.77 - 5.03 | 3.88 - 4.00 |
| Process time [min] | 690 | 135 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

At the end of the functional coating 4 % and at the end of the top coating process 7 - 9% (n=3) of the batch showed bridging.

### Disintegration Test (according to European Pharmacopeia 2.9.1 Test B modified method based on gastro-resistant capsules)

| | |
|---|---|
| Sample preparation: | Samples will be filled with 500 mg of a Lactose/Caffeine (containing either 20 mg or 200 mg Caffeine) blend and length of the capsule will be documented (target 21.7 + 0.3 mm). |
| Method: | 2h 0.1 N HCl followed by full by 30 minutes phosphate buffer pH 5.5 followed by 1 hour phosphate buffer pH 6.8. |
| Detection method: | Visually and electrical Impedance |
| Temperature: | 37.0°C |
| Media I: | 700 ml 0.1 N HCl according to European Pharmacopeia without disks |
| Media II: | 700mL phosphate buffer pH 5.5 without disks |
| Media III: | 700mL phosphate buffer pH 6.8 without disks |
| Samples: | n=6 |

Composition phosphate buffer pH 5.5 (Media II):
2100 ml 0.1 N HCl + 534 g 0.2 M Na₃PO₄ aq. with 2N HCl or 2N NaOH adjusted to pH 5.50 (± 0,05).

Composition phosphate buffer pH 6.8 (Medium III):
2100 ml 0.1 N HCl + 684 g 0.2 M Na₃PO₄ aq. with 2 N HCl or 2 N NaOH adjusted to pH 6,80 (± 0,05).

**Table 24: Disintegration Results**

| Media | Time [min] | Sub Sample 1 n=3 | Sub Sample 2 n=3 |
|---|---|---|---|
| 0.1 N HCl | Start | intact | intact |
| 0.1 N HCl | 120 minutes | intact | intact |
| pH 5.5 | 30 minutes | intact | intact |
| pH 6.8 | < 60 minutes | release | release |

### Example 9: (Inventive) Enteric coating of pre-locked capsules in drum coater

The functional and top coat formulations are calculated considering a surface area in pre-locked state of 545.8 mm² and a batch size of 40,000 capsules (Vcaps plus Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 25: Functional Coating - Formulation 2 of Example 1**

| **Material** | **Composition** | **Solid Composition Percentage** |
|---|---|---|
| EUDRAGIT^{®} L 30 D-55 | 3.15 mg/cm² | 67.2 % |
| EUDRAGIT^{®} NM 30 D | 0.35 mg/cm² | 7.5 % |
| Triethyl citrate | 20.0 % on ds* | 14.9 % |
| Glycerol monostearate | 10.0 % on ds* | 3.0 % |
| Polysorbate 80 | 4.0 % on ds* | 7.5 % |
| Demineralized Water | On demand | n/a |
| | | |
| Solid content | 10.0% w/w | |
| Total solid weight gain | 4.7 mg/cm² | |
| Total applied polymer | 3.5 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 26: Top Coating - Formulation 5 of Example 1**

| **Material** | **Composition** | **Solid Composition Percentage** |
|---|---|---|
| METHOCEL^{™} VLV | 0.45 mg/cm² | 90.9 % |
| Glycerol monostearate | 5.9 % on ds* | 5.4 % |
| Triethyl citrate | 2.9 % on ds* | 2.6 % |
| Polysorbat 80 | 1.2 % on ds* | 1.1 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules are coated in a fully perforated side-vended pan coating system Bohle BFC 40. The relevant process parameters are listed in Table .

The equipment parameters were kept equal for functional and top coating.

**Table 27: Process Parameter**

| Parameter | **Value** Functional Coating | **Value** Top Coating |
|---|---|---|
| Machine | Bohle BFC 40 | |
| Batch size [kg] | 3.84 | |
| Nozzle bore [mm] | 1.0 | |
| Number of Nozzles | 3 | |
| Spray Gun to Product Bed Distances [cm] | 20 | |
| Spray rate [g/min] | 30 - 45 | 30-40 |
| Exhaust air temperature [°C] | 30 | 30 |
| Inlet air temperature [°C] | 37 - 44 | 36-44 |
| Inlet air volume [m³/h] | 500 | 450 - 500 |
| Atomizing pressure [bar] | 1.0 | 1.0 |
| Flat pattern pressure [bar] | 1.2 | 1.2 |
| Pan speed [rpm] | 8 | 8 |
| Product temperature [°C] | 29 - 31 | 30 |
| LOD [%] | 3.84 - 4.64 | 3.47 - 3,74 |
| Process time [min] | 355 | 45 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested. According to the present invention less than 10% of capsules of the batch showing bridging is considered acceptable.

### Results:

At the end of the functional coating 3 % and at the end of the top coating process 3% (n=3) of the batch showed bridging.

### Disintegration Test (according to European Pharmacopeia 2.9.1 Test B modified method based on gastro-resistant capsules)

| | |
|---|---|
| Sample preparation: | Samples will be filled with 500 mg of a Lactose/Caffeine (containing either 20 mg or 200 mg Caffeine) blend and length of the capsule will be documented (target 21.7 + 0.3 mm). |
| Method: | 2h 0.1 N HCl followed by full by 30 minutes phosphate buffer pH 5.5 followed by 1 hour phosphate buffer pH 6.8. |
| Detection method: | Visually and electrical Impedance |
| Temperature: | 37.0°C |
| Media I: | 700 ml 0.1 N HCl according to European Pharmacopeia without disks |
| Media II: | 700mL phosphate buffer pH 5.5 without disks |
| Media III: | 700mL phosphate buffer pH 6.8 without disks |
| Samples: | n=6 |

Composition phosphate buffer pH 5.5 (Media II):
2100 ml 0.1 N HCl + 534 g 0.2 M Na₃PO₄ aq. with 2N HCl or 2N NaOH adjusted to pH 5.50 (± 0.05).

Composition phosphate buffer pH 6.8 (Medium III):
2100 ml 0.1 N HCl + 684 g 0.2 M Na₃PO₄ aq. with 2 N HCl or 2 N NaOH adjusted to pH 6.80 (± 0.05).

**Table 28: Disintegration Results**

| Media | Time [min] | Sub Sample 1 n=3 | Sub Sample 2 n=3 |
|---|---|---|---|
| 0.1 N HCl | Start | intact | intact |
| 0.1 N HCl | 120 minutes | intact | intact |
| pH 5.5 | 30 minutes | intact | intact |
| pH 6.8 | < 60 minutes | release | release |

### Dissolution Test (according to European Pharmacopeia (2.9.3) apparatus II)

Capsule manually filled. The polymer coated pre-locked capsules were manually filled with 500mg Caffeine/Lactose Mixture 4:6, closed to the final-locked state and tested in a dissolution test.

| | |
|---|---|
| Method: | |
| Apparatus: | ERWEKA DT 700 Paddle Apparatus (USP II) |
| Detection method: | Online UV |
| Temperature: | 37.5°C |
| Media I: | 700 ml 0.1 N HCl according to European Pharmacopeia without disks |
| Media II: | 700mL phosphate buffer pH 5.5 without disks |
| Media III: | 700mL phosphate buffer pH 6.8 without disks |
| Samples: | n=6 |
| Paddle speed: | 75rpm |

Composition phosphate buffer pH 5.5 (Media II):
2100 ml 0.1 N HCl + 534 g 0.2 M Na₃PO₄ aq. with 2N HCl or 2N NaOH adjusted to pH 5.50 (± 0,05).

Composition phosphate buffer pH 6.8 (Medium III):
2100 ml 0.1 N HCl + 684 g 0.2 M Na₃PO₄ aq. with 2 N HCl or 2 N NaOH adjusted to pH 6,80 (± 0,05).

**Table 29: Dissolution Results**

| Media | Time [min] | Sample 1 [% released] | Sample 2 [% released] | Sample 3 [% released] | Sample 4 [% released] | Sample 5 [% released] | Sample 6 [% released] |
|---|---|---|---|---|---|---|---|
| 0.1 N HCl | 0 | 0.00 | 0.00 | 0.01 | 0.01 | 0.01 | 0.00 |
| 0.1 N HCl | 60 | 0.00 | 0.00 | 0.02 | 0.02 | 0.01 | 0.00 |
| 0.1 N HCl | 120 | 0.00 | 0.00 | 0.01 | 0.00 | 0.02 | 0.00 |
| pH 5.5 | 150 | 0.00 | 0.01 | 0.01 | 0.04 | 0.05 | 0.01 |
| pH 6.2 | 165 | 0.56 | 2.55 | 4.54 | 0.41 | 2.95 | 18.63 |
| pH 6.2 | 180 | 77.02 | 95.74 | 74.20 | 93.00 | 98.43 | 99.58 |
| pH 6.2 | 195 | 98.18 | 100.66 | 99.72 | 102.01 | 99.72 | 101.34 |
| pH 6.2 | 210 | 98.92 | 100.76 | 100.82 | 101.75 | 99.81 | 101.39 |
| pH 6.8 | 225 | 97.01 | 98.64 | 98.94 | 99.70 | 97.73 | 99.52 |
| pH 6.8 | 240 | 97.04 | 98.71 | 98.81 | 99.91 | 97.82 | 99.67 |
| pH 6.8 | 255 | 97.04 | 98.97 | 98.85 | 99.76 | 97.83 | 99.31 |
| pH 6.8 | 270 | 97.11 | 98.82 | 98.90 | 100.00 | 97.88 | 99.46 |

Example 10 (comparative) according to WO 2020/229178: Enteric coating of Standard EUDRAGIT^{®} L 30D-55 coating with Glycerol mono stearate (GMS) on pre-locked capsules followed by top coat of HPMC in drum coater and automatic capsule filling GMS emulsion was prepared by adding Polysorbate 80 (33% solution), Triethyl citrate and GMS in hot water (70-80 °C) under high shear homogenizer for 10 minutes. Prepared GMS emulsion was allowed to cool down at room temperature and then added to EUDRAGIT^{®} polymer dispersion under overhead stirring. The spraying suspension was gently stirred during the coating process. The capsules were coated in the pre-locked state utilizing a drum coater. Top coat: HPMC was dissolved in water under stirring and sprayed on to the coated capsules utilizing a drum coater.

**Table 30: formulation in accordance to comparative example 7 of WO 2020/229178.**

| **Material** | **Composition** | **Solid Composition Percentage** |
|---|---|---|
| EUDRAGIT^{®} L 30 D-55 | 2 mg/cm² | 85.47 % |
| Triethyl citrate | 10.0 % on ds* | 8.55 % |
| Glycerol monostearate | 5.0 % on ds* | 4.27 % |
| Polysorbate 80 (33% solution) | 2.0 % on ds* | 1.71 % |
| Demineralized Water | On demand | n/a |
| | | |
| Solid content | 10.0% w/w | |
| Total solid weight gain | 2.34 mg/cm² | |
| Surface Tension mN/m ( average of 2 measures) DIN EN 14370:2004 | 46.52 mN/m | |

## Claims

1. Process for preparing a polymer-coated hard shell capsule, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and coated with a coating solution, suspension or dispersion comprising or consisting of
a) at least one polymer;
b) at least one glidant;
c) at least one emulsifier;
d) optionally at least one plasticizer;
e) optionally at least one biologically active ingredient; and
f) optionally at least one additive, different from a) to e);
to obtain a coated, preferably only at the outer surface, hard shell capsule in the pre-locked state, wherein the coating solution, suspension or dispersion has a surface tension of at most 38 mN/m as measured in accordance to DIN EN 14370:2004.

2. The process according to claim 1, wherein the base material of the body and the cap is selected from hydroxypropyl methyl cellulose, starch, gelatin, pullulan and a copolymer of a C1- to C4-alkylester of (meth)acrylic acid and (meth)acrylic acid.

3. The process according to any of the preceding claims, wherein the at least one polymer is selected from at least one (meth)acrylate copolymer.

4. the process according to claim 1 or 2, wherein the at least one polymer is selected from at least one anionic cellulose, ethyl cellulose or starch comprising at least 35 % by weight amylose.

5. The process according to any of the preceding claims, wherein the at least one polymer is
I. a core-shell polymer, which is a copolymer obtained by a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid; or
II. an anionic polymer obtained by polymerizing 25 to 95 % by weight C1- to C12-alkyl esters of acrylic acid or of methacrylic acid and 75 to 5% by weight (meth)acrylate monomers with an anionic group; or
III. a cationic (meth)acrylate copolymer obtained by polymerizing C1- to C4-alkyl esters of acrylic or of methacrylic acid and an alkyl ester of acrylic or of methacrylic acid with a tertiary or a quaternary ammonium group in the alkyl group; or
iv) a (meth)acrylate copolymer obtained by polymerizing methacrylic acid and ethyl acrylate, methacrylic acid and methyl methacrylate, ethyl acrylate and methyl methacrylate or methacrylic acid, methyl acrylate and methyl methacrylate; or
v) a (meth)acrylate copolymer obtained by polymerizing 40 to 60 % by weight of methacrylic acid and 60 to 40 % by weight of ethyl acrylate; or
vi) a (meth)acrylate copolymer obtained by polymerizing 60 to 80 % of ethyl acrylate and 40 to 20 % by weight of methyl methacrylate; or
vii) a (meth)acrylate copolymer obtained by polymerizing 5 to 15 % by weight methacrylic acid, 60 to 70 % by weight of methyl acrylate and 20 to 30 % by weight methyl methacrylate; or mixtures thereof.

6. The process according to any of the preceding claims, wherein the at least one polymer is a mixture of
i) a (meth)acrylate copolymer obtained by copolymerizing 40 to 60 % by weight of methacrylic acid and 60 to 40 % by weight of ethyl acrylate and a (meth)acrylate copolymer obtained by polymerizing 60 to 80 % of ethyl acrylate and 40 to 20 % by weight of methyl methacrylate at a ratio from 10:1 to 1:10 by weight; or
ii) a (meth)acrylate copolymer obtained by copolymerizing 5 to 15 % by weight methacrylic acid, 60 to 70 % by weight of methyl acrylate and 20 to 30 % by weight methyl methacrylate and a (meth)acrylate copolymer obtained by copolymerizing 40 to 60 % by weight of methacrylic acid and 60 to 40 % by weight of ethyl acrylate at a ratio from 1:1 to 5:1 by weight.

7. the process according to any of the preceding claims, wherein the at least one glidant
i) is present in an amount of 3 to 75 % by weight, based on the total weight of the at least one polymer a) and/or
ii) is selected from silica, ground silica, fumed silica, kaolin calcium silicate, magnesium silicate, colloidal silicone dioxide, talc, stearate salts, sodium stearyl fumarate, starch, stearic acid and glycerol monostearate or mixtures thereof.

8. The process according to any of the preceding claims, wherein the at least one emulsifier
i) is present in an amount of 1.5 to 40 % by weight, based on the total weight of the at least one polymer a) and/or
ii) is a non-ionic emulsifier.

9. The process according to any of the preceding claims, wherein the at least one plasticizer
i) is present in an amount of 2 to 40 % by weight, based on the total weight of the at least one polymer a) and/or
ii) is selected from alkyl citrates, alkyl phthalates, and alkyl sebacates, polyethylene glycols, propylene glycols or combinations thereof.

10. The process according to any of the preceding claims, wherein up to 400 % by weight, on the total weight of the at least one polymer a) of at least one additive are comprised.

11. The process according to any of the preceding claims, wherein the body and the cap are comprising encircling notches or dimples in the area where the cap overlaps the body, that allow the capsule to be closed by a snap-into-place mechanism either in the pre-locked state or in the final-locked state.

12. The process according to any of the preceding claims, wherein the body comprises a tapered rim.

13. The process according to any of the preceding claims, wherein the coating layer is applied in an amount of about 0.7 to 20 mg/cm².

## Patentansprüche

1. Verfahren zur Herstellung einer polymerbeschichteten Hartschalenkapsel, die als Behälter für pharmazeutische oder nutrazeutische biologische Wirkstoffe geeignet ist, wobei die Hartschalenkapsel einen Körper und eine Kappe umfasst, wobei die Kappe im verschlossenen Zustand den Körper entweder in einem vorverriegelten Zustand oder in einem endverriegelten Zustand überlappt, wobei die Hartschalenkapsel im vorverriegelten Zustand bereitgestellt wird und mit einer Beschichtungslösung, -suspension oder -dispersion beschichtet ist, die Folgendes umfasst oder daraus besteht:
a) mindestens ein Polymer;
b) mindestens ein Gleitmittel;
c) mindestens einen Emulgator;
d) gegebenenfalls mindestens einen Weichmacher;
e) gegebenenfalls mindestens einen biologischen Wirkstoff und
f) gegebenenfalls mindestens ein von a) bis e) verschiedenes Additiv;
wodurch eine vorzugsweise nur an der äußeren Oberfläche beschichtete Hartschalenkapsel im vorverriegelten Zustand erhalten wird, wobei die Beschichtungslösung, - suspension oder -dispersion eine gemäß DIN EN 14370:2004 gemessene Oberflächenspannung von höchstens 38 mN/m aufweist.

2. Verfahren nach Anspruch 1, wobei das Ausgangsmaterial des Körpers und der Kappe aus Hydroxypropylmethylcellulose, Stärke, Gelatine, Pullulan und einem Copolymer eines C1- bis C4-Alkylesters von (Meth)Acrylsäure und (Meth)Acrylsäure ausgewählt ist.

3. Verfahren nach einem der vorhergehenden Ansprüche, wobei das mindestens eine Polymer aus mindestens einem (Meth)Acrylat-Copolymer ausgewählt ist.

4. Verfahren nach Anspruch 1 oder 2, wobei das mindestens eine Polymer aus mindestens einer anionischen Cellulose, Ethylcellulose oder Stärke, die mindestens 35 Gew.-% Amylose umfasst, ausgewählt ist.

5. Verfahren nach einem der vorhergehenden Ansprüche, wobei das mindestens eine Polymer Folgendes ist:
I. ein Kern-Schale-Polymer, bei dem es sich um ein Copolymer handelt, das durch ein zweistufiges Emulsionspolymerisationsverfahren erhalten wird, mit einem Kern mit 70 bis 80 Gew.-%, der polymerisierte Einheiten aus 65 bis 75 Gew.-% Ethylacrylat und 25 bis 35 Gew.-% Methylmethacrylat umfasst, und einer Schale mit 20 bis 30 Gew.-%, die polymerisierte Einheiten aus 45 bis 55 Gew.-% Ethylacrylat und 45 bis 55 Gew.-% Methacrylsäure umfasst; oder
II. ein anionisches Polymer, das erhalten wird, indem 25 bis 95 Gew.-% C1- bis C12-Alkylester von Acrylsäure oder Methacrylsäure und 75 bis 5 Gew.-% (Meth)AcrylatMonomere mit einer anionischen Gruppe polymerisiert werden; oder
III. ein kationisches (Meth)Acrylat-Copolymer, das erhalten wird, indem C1- bis C4-Alkylester von Acryl- oder Methacrylsäure und ein Alkylester von Acryl- oder Methacrylsäure mit einer tertiären oder einer quartären Ammoniumgruppe in der Alkylgruppe polymerisiert werden; oder
iv) ein (Meth)Acrylat-Copolymer, das erhalten wird, indem Methacrylsäure und Ethylacrylat, Methacrylsäure und Methylmethacrylat, Ethylacrylat und Methylmethacrylat oder Methacrylsäure, Methylacrylat und Methylmethacrylat polymerisiert werden; oder
v) ein (Meth)Acrylat-Copolymer, das erhalten wird, indem 40 bis 60 Gew.-% Methacrylsäure und 60 bis 40 Gew.-% Ethylacrylat polymerisiert werden; oder
vi) ein (Meth)Acrylat-Copolymer, das erhalten wird, indem 60 bis 80 Gew.-% Ethylacrylat und 40 bis 20 Gew.-% Methylmethacrylat polymerisiert werden; oder
vii) ein (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat polymerisiert werden; oder Mischungen davon.

6. Verfahren nach einem der vorhergehenden Ansprüche, wobei das mindestens eine Polymer eine Mischung von Folgendem ist:
i) einem (Meth)Acrylat-Copolymer, das erhalten, wird, indem 40 bis 60 Gew.-% Methacrylsäure und 60 bis 40 Gew.-% Ethylacrylat copolymerisiert werden, und einem (Meth)Acrylat-Copolymer, das erhalten, wird, indem 60 bis 80 Gew.-% Ethylacrylat und 40 bis 20 Gew.-% Methylmethacrylat in einem Gewichtsverhältnis von 10:1 bis 1:10 polymerisiert werden; oder
ii) einem (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat copolymerisiert werden, und einem (Meth)Acrylat-Copolymer, das erhalten wird, indem 40 bis 60 Gew.-% Methacrylsäure und 60 bis 40 Gew.-% Ethylacrylat in einem Gewichtsverhältnis von 1:1 bis 5:1 copolymerisiert werden.

7. Verfahren nach einem der vorhergehenden Ansprüche, wobei das mindestens eine Gleitmittel
i) in einer Menge von 3 bis 75 Gew.-%, bezogen auf das Gesamtgewicht des mindestens einen Polymers a), vorliegt und/oder
ii) aus Siliciumdioxid, gemahlenem Siliciumdioxid, Quarzstaub, Kaolin, Calciumsilicat, Magnesiumsilicat, kolloidalem Siliciumdioxid, Talk, Stearatsalzen, Natriumstearylfumarat, Stärke, Stearinsäure und Glycerinmonostearat oder Mischungen davon ausgewählt ist.

8. Verfahren nach einem der vorhergehenden Ansprüche, wobei der mindestens eine Emulgator
i) in einer Menge von 1,5 bis 40 Gew.-%, bezogen auf das Gesamtgewicht des mindestens einen Polymers a), vorliegt und/oder
ii) ein nichtionischer Emulgator ist.

9. Verfahren nach einem der vorhergehenden Ansprüche, wobei der mindestens eine Weichmacher
i) in einer Menge von 2 bis 40 Gew.-%, bezogen auf das Gesamtgewicht des mindestens einen Polymers a), vorliegt und/oder
ii) aus Alkylcitraten, Alkylphthalaten und Alkylsebacaten, Polyethylenglycolen, Propylenglycolen oder Kombinationen davon ausgewählt ist.

10. Verfahren nach einem der vorhergehenden Ansprüche, wobei bis zu 400 Gew.-%, bezogen auf das Gesamtgewicht des mindestens einen Polymers a), mindestens eines Additivs enthalten sind.

11. Verfahren nach einem der vorhergehenden Ansprüche, wobei der Körper und die Kappe umlaufende Aussparungen oder Vertiefungen in dem Bereich umfassen, in dem die Kappe den Körper überlappt, die ein Verschließen der Kapsel durch einen Einrastmechanismus entweder in den vorverriegelten Zustand oder in den endverriegelten Zustand ermöglichen.

12. Verfahren nach einem der vorhergehenden Ansprüche, wobei der Körper einen konischen Rand umfasst.

13. Verfahren nach einem der vorhergehenden Ansprüche, wobei die Überzugsschicht in einer Menge von etwa 0,7 bis 20 mg/cm² aufgetragen wird.

## Revendications

1. Procédé de préparation d'une capsule à enveloppe dure enrobée de polymère adaptée en tant que contenant pour des principes actifs pharmaceutiques ou nutraceutiques biologiquement actifs, dans lequel la capsule à enveloppe dure comprend un corps et un capuchon, dans lequel, à l'état fermé, le capuchon chevauche le corps soit dans un état préverrouillé, soit dans un état verrouillé final, dans lequel la capsule à enveloppe dure est fournie à l'état préverrouillé et enrobée avec une solution, suspension ou dispersion d'enrobage comprenant ou constituée par
a) au moins un polymère ;
b) au moins un agent glissant ;
c) au moins un émulsifiant ;
d) facultativement au moins un plastifiant ;
e) facultativement au moins un composant biologiquement actif ; et
f) facultativement au moins un additif, différent de a) à e) ;
pour obtenir une capsule à enveloppe dure enrobée, de préférence uniquement sur la surface externe, à l'état préverrouillé, la solution, suspension ou dispersion d'enrobage ayant une tension superficielle d'au plus 38 mN/m telle que mesurée selon la norme DIN EN 14370:2004.

2. Procédé selon la revendication 1, dans lequel le matériau de base du corps et du capuchon est choisi parmi l'hydroxypropylméthylcellulose, l'amidon, la gélatine, le pullulane et un copolymère d'un ester d'alkyle en C1 à C4 d'acide (méth)acrylique et d'acide (méth)acrylique.

3. Procédé selon l'une quelconque des revendications précédentes, dans lequel le ou les polymères sont choisis parmi au moins un copolymère de (méth)acrylate.

4. Procédé selon la revendication 1 ou 2, dans lequel le ou les polymères sont choisis parmi au moins une cellulose anionique, une éthylcellulose ou un amidon comprenant au moins 35 % en poids d'amylose.

5. Procédé selon l'une quelconque des revendications précédentes, dans lequel le ou les polymères sont
I. un polymère noyau-enveloppe, qui est un copolymère obtenu par un procédé de polymérisation en émulsion en deux étapes avec un noyau représentant 70 à 80 % en poids, comprenant des motifs polymérisés de 65 à 75 % en poids d'acrylate d'éthyle et de 25 à 35 % en poids de méthacrylate de méthyle, et une enveloppe représentant 20 à 30 % en poids, comprenant des motifs polymérisés de 45 à 55 % en poids d'acrylate d'éthyle et de 45 à 55 % en poids d'acide méthacrylique ; ou
II. un polymère anionique obtenu par polymérisation de 25 à 95 % en poids d'esters d'alkyle en C1 à C12 d'acide acrylique ou d'acide méthacrylique et de 75 à 5 % en poids de monomères de (méth)acrylate comportant un groupement anionique ; ou
III. un copolymère de (méth)acrylate cationique obtenu par polymérisation d'esters d'alkyle en C1 à C4 d'acide acrylique ou méthacrylique et d'un ester d'alkyle d'acide acrylique ou d'acide méthacrylique comportant un groupe ammonium tertiaire ou un groupe ammonium quaternaire dans le groupe alkyle ; ou
iv) un copolymère de (méth)acrylate obtenu par polymérisation d'acide méthacrylique et d'acrylate d'éthyle, d'acide méthacrylique et de méthacrylate de méthyle, d'acrylate d'éthyle et de méthacrylate de méthyle ou d'acide méthacrylique, d'acrylate de méthyle et de méthacrylate de méthyle ; ou
v) un copolymère de (méth)acrylate obtenu par polymérisation de 40 à 60 % en poids d'acide méthacrylique et de 60 à 40 % en poids d'acrylate d'éthyle ; ou
vi) un copolymère de (méth)acrylate obtenu par polymérisation de 60 à 80 % d'acrylate d'éthyle et de 40 à 20 % en poids de méthacrylate de méthyle ; ou
vii) un copolymère de (méth)acrylate obtenu par polymérisation de 5 à 15 % en poids d'acide méthacrylique, de 60 à 70 % en poids d'acrylate de méthyle et de 20 à 30 % en poids de méthacrylate de méthyle ; ou des mélanges de ceux-ci.

6. Procédé selon l'une quelconque des revendications précédentes, dans lequel le ou les polymères sont un mélange de
i) un copolymère de (méth)acrylate obtenu par copolymérisation de 40 à 60 % en poids d'acide méthacrylique et de 60 à 40 % en poids d'acrylate d'éthyle et un copolymère de (méth)acrylate obtenu par polymérisation de 60 à 80 % en poids d'acrylate d'éthyle et de 40 à 20 % en poids de méthacrylate de méthyle à un rapport de 10:1 à 1:10 en poids ; ou
ii) un copolymère de (méth)acrylate obtenu par copolymérisation de 5 à 15 % en poids d'acide méthacrylique, de 60 à 70 % en poids d'acrylate de méthyle et de 20 à 30 % en poids de méthacrylate de méthyle et un copolymère de (méth)acrylate obtenu par copolymérisation de 40 à 60 % en poids d'acide méthacrylique et de 60 à 40 % en poids d'acrylate d'éthyle à un rapport de 1:1 à 5:1 en poids.

7. Procédé selon l'une quelconque des revendications précédentes, dans lequel le ou les agents glissants i) sont présents en une quantité de 3 à 75 % en poids, par rapport au poids total du ou des polymères a) et/ou ii) sont choisis parmi la silice, la silice broyée, la silice pyrogénée, le kaolin, le silicate de calcium, le silicate de magnésium, le dioxyde de silicium colloïdal, le talc, des sels de stéarates, le stéarylfumarate de sodium, l'amidon, l'acide stéarique, le monostéarate de glycérol ou des mélanges de ceux-ci.

8. Procédé selon l'une quelconque des revendications précédentes, dans lequel le ou les émulsifiants i) sont présents en une quantité de 1,5 à 40 % en poids, par rapport au poids total du ou des polymères a) et/ou ii) est un émulsifiant non ionique.

9. Procédé selon l'une quelconque des revendications précédentes, dans lequel le ou les plastifiants i) sont présents en une quantité de 2 à 40 % en poids, par rapport au poids total du ou des polymères a) et/ou ii) est choisi parmi les citrates d'alkyle, les phtalates d'alkyle, les sébacates d'alkyle, les polyéthylèneglycols, les propylèneglycols ou des combinaisons de ceux-ci.

10. Procédé selon l'une quelconque des revendications précédentes, dans lequel jusqu'à 400 % en poids, par rapport au poids total du ou des polymères a), d'au moins un additif sont inclus.

11. Procédé selon l'une quelconque des revendications précédentes, dans lequel le corps et le capuchon comprennent des encoches ou des creux d'encerclement dans la zone où le capuchon chevauche le corps, qui permettent de fermer la capsule au moyen d'un mécanisme d'encliquetage soit dans l'état préverrouillé, soit dans l'état verrouillé final.

12. Procédé selon l'une quelconque des revendications précédentes, dans lequel le corps comprend un rebord effilé.

13. Procédé selon l'une quelconque des revendications précédentes, dans lequel la couche d'enrobage est appliquée en une quantité d'environ 0,7 à 20 mg/cm².
